(19) Europäisches Patentamt
European Patent Office
Office européen des brevets

(11) EP 2 848 693 A1

(12) EUROPÄISCHE PATENTANMELDUNG

(43) Veröffentlichungstag:
**18.03.2015 Patentblatt 2015/12**

(51) Int Cl.:
*C12P 5/00* (2006.01) *C12P 9/00* (2006.01)

(21) Anmeldenummer: **13184167.8**

(22) Anmeldetag: **12.09.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **LANXESS Deutschland GmbH**
**50569 Köln (DE)**

(72) Erfinder:
- **Keim, Jeanette**
  **06110 Halle (DE)**
- **Ludwig, Steve**
  **06110 Halle (DE)**
- **Schreckenbach, Hans Felix**
  **06114 Halle (DE)**
- **Früh, Thomas**
  **42105 Wuppertal (DE)**
- **Dreisbach, Claus**
  **42799 Leichlingen (DE)**
- **Wessjohann, Ludger**
  **06120 Halle (DE)**

(74) Vertreter: **Godemeyer Blum Lenze Patentanwälte Partnerschaft mbB**
**werkpatent**
**An den Gärten 7**
**51491 Overath (DE)**

(54) **Verkettung von halogenierten Alkenyldiphosphat-Derivaten**

(57) Die Erfindung betrifft ein Verfahren zur enzymatischen Verkettung von Monomeren Verfahren zur enzymatischen Verkettung von Monomeren enthaltend die Schritte:
a) Verketten von Monomeren mit der Formel (I)

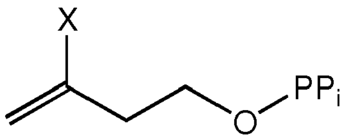

(I),

wobei X ein Halogenatom und O-$PP_i$ eine Diphosphatgruppe ist, und optional von Isopentenyldiphosphat als weiterem Monomer,
wobei die Monomere mit einer Prenyltransferase und einem allylischen Diphosphat als Startersubstrat in Anwesenheit zweiwertiger Ionen in Kontakt gebracht werden;
b) Erhalten von Produkten mit Molekülketten, die Monomereinheiten enthalten, die durch die Formel (II) dargestellt werden:

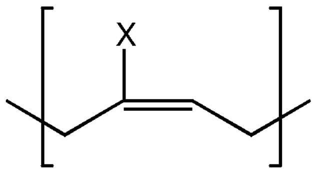

(II)

wobei X wie oben definiert ist; und
optional, wobei die Molekülketten zusätzlich Monomereinheiten enthalten, die durch die Formel (III) dargestellt werden:

EP 2 848 693 A1

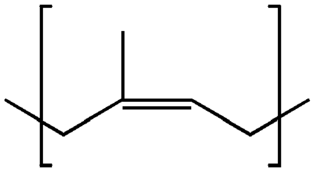 (III).

Die Erfindung betrifft weiterhin Produkte mit der Formel (IV)

$R^1$-$A_n$-$B_m$-O-$PP_i$ (IV),

wobei $R^1$ der Rest eines allylischen Startersubstrats ist, A die Monomereinheit [$CH_2$-C(X)=CH-$CH_2$] und B die Monomereinheit [$CH_2$-C($CH_3$)=CH-$CH_2$] darstellt,
wobei X ein Halogenatom, O-$PP_i$ eine Diphosphatgruppe oder durch O-H ersetzt ist;
und Produkte mit der Formel (V)

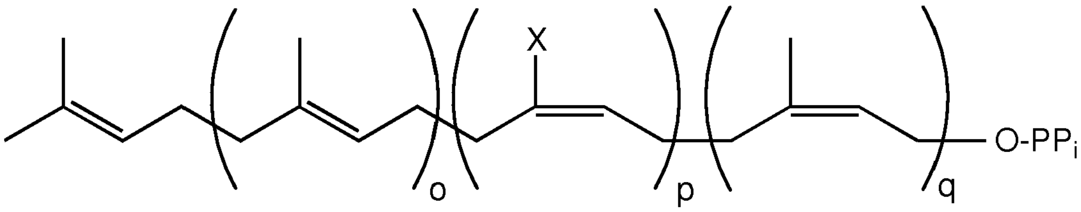

(V),

und der Formel (VI)

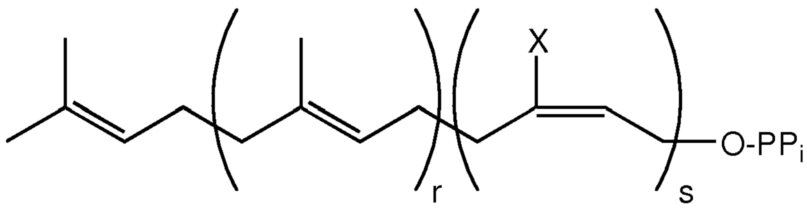

(VI),

wobei X ein Halogenatom, O-$PP_i$ eine Diphosphatgruppe oder durch O-H ersetzt ist, o und r eine ganze Zahl von 1 bis 6, p und s eine ganze Zahl von 1 bis 30000 ist, und q eine ganze Zahl von 0 bis 30000 ist.

Die Erfindung betrifft auch die Verwendung von Prenyltransferasen zur Herstellung dieser Produkte.

**Beschreibung**

Die vorliegende Erfindung betrifft ein Verfahren zur enzymatischen Verkettung von halogenierten Alkenyldiphosphat-Derivaten, ein Verfahren zur enzymatischen Herstellung von Produkten, insbesondere Polymeren und Copolymeren auch zusammen mit nicht-halogenierten Derivaten, enthaltend diese Monomereinheiten, sowie entsprechende Polymere oder Copolymere.

Hintergrund der Erfindung

Natürlicher Kautschuk wird hauptsächlich aus dem Kautschukbaum *Hevea brasiliensis* gewonnen und stellt ein einzigartiges hochmolekulares Biopolymer dar. Durch Vulkanisieren des natürlichen Kautschuks entsteht ein sehr elastischer und widerstandsfähiger Kautschuk, der Anwendung in vielen Bereichen der Gummiherstellung findet, wie etwa in Autoreifen, Gummihandschuhe, Luftballons, Latexkleidung, usw. Neben dem Kautschukbaum *Hevea brasiliensis* als Rohstoffquelle für natürlichen Kautschuk gibt es aber auch Bestrebungen, größere Mengen Milchsaft (Latex) aus dem russischen Löwenzahn *Taraxacum koksaghyz* zu gewinnen. Dies liegt darin begründet, dass besonders das vermehrte Auftreten von Allergien gegenüber Proteinen des Kautschukbaums die Verwendung des natürlichen Materials immer schwieriger macht.

Natürlicher Kautschuk besteht aus 1,4-verknüpften Isopreneinheiten in *cis*-Konfiguration mit einem Molekulargewicht von etwa $10^6$ g $mol^{-1}$, daneben enthält natürlicher Kautschuk aber auch noch einen geringen Anteil (<1 %) anderer Substanzen, die für spezielle Eigenschaften dieses Polymers verantwortlich sind. Die Polymermoleküle des Naturkautschuks werden in den Latex-Gefäßen (Latiziferen) des Kautschukbaums produziert und aggregiert, wobei Gummipartikel erhalten werden. Die Biosynthese von Naturkautschuk erfolgt fast vollständig aus von Isopentenyldiphosphat (auch als Isopentenylpyrophosphat oder abgekürzt als "IPP" bezeichnet) stammenden Isopreneinheiten. Als Startersubstrat wird ein allylisches Prenyldiphosphat benötigt, damit die nachfolgende Kettenverlängerungsreaktion zur Bildung der Kautschukmakromoleküle initiiert werden kann. Die Synthese der allylischen Prenyldiphosphate aus Isopentenyldiphosphat (IPP) wird durch die IPP-Isomerase und Prenyltransferase katalysiert. Isopentenyldiphosphat (IPP) und das aus dieser Verbindung durch Isomerisierung hergestellte Dimethylallyldiphosphat (DMAPP) wird durch den Mevalonatbiosyntheseweg im Cytosol synthetisiert. Daneben wurde ein zweiter Stoffwechselweg zum IPP und DMAPP entdeckt, der sogenannte MEP-Weg (Methylerythritolphosphatweg). Dieser Stoffwechselweg ist unter anderem in den pflanzlichen Plastiden und in dem Erreger der Malaria zu finden.

Das Enzym, das für die *cis*-1,4-Polymerisation der aus IPP stammenden Isopreneinheiten an das allylische Startermolekül verantwortlich ist, ist die an Kautschukpartikel gebundene Kautschuk-Synthase ("Rubber-Synthase"; EC 2.5.1.20). Dieses Enzym kann die Synthese hochmolekularer Polyisoprene von über $C_{100000}$ realisieren.

Neben der Kautschuk-Synthase gibt es noch eine Vielzahl anderer Prenyltransferasen, die die Ausgangsstoffe für die Naturstoffe der Terpene und Terpenoide synthetisieren. Den Terpenen und dem Kautschuk ist gemeinsam, dass sie sich auf das Gerüst des ungesättigten Kohlenwasserstoffs Isopren zurückführen lassen. Mit bislang ca. 40.000 beschriebenen Substanzen bilden Isoprenoide die größte Klasse sekundärer Pflanzenstoffe. In Pflanzen haben sie vielfältige Funktionen und sind an unterschiedlichen Prozessen beteiligt. Für industrielle Anwendungen sind Isoprenoide von besonderem Interesse.

Die Einteilung der Prenyltransferasen, die Polyprenyldiphosphate synthetisieren, erfolgt grundsätzlich nach den synthetisierten Produkten, in *cis*- und *trans*-Prenyltransferasen. Dabei unterscheidet man je nach Kettenlänge des entstehenden Produktes zwischen Kurz-, Mittel- und Langketten-Prenyltransferasen.

Wie bereits oben erläutert, ist eine enzymatische Synthese von Kautschuk aus dem Gummibaum *Hevea brasiliensis* bereits bekannt, allerdings konnte bisher für die Kautschuk-Synthase nur eine Aktivität mit Isopentenyldiphosphat (IPP) nachgewiesen werden. Die Arbeit mit der Kautschuk-Synthase aus dem Gummibaum *Hevea brasiliensis* wird durch den Umstand, dass sie kaum heterolog exprimierbar und zudem auf Helferproteine angewiesen ist, besonders erschwert. Eine kombinierte chemische und enzymatische Synthese könnte daher die Herstellung neuartiger und auch bisher chemisch synthetisierter Gummiarten unterstützen. Insbesondere sind auch artifizielle Verlängerungssubstrate für die Prenyltransferasen interessant, weil in speziellen synthetisch erzeugten Gummiarten strukturell veränderte Polyisoprene vorkommen. Ein Beispiel hierfür ist ChloroprenKautschuk (auch Polychloropren oder Chlorbutadien-Kautschuk), der unter anderem z.B. unter der Bezeichnung Neopren® vertrieben wird. Die Herstellung dieses Synthesekautschuks erfolgt durch Polymerisation von 2-Chlor-1,3-butadien (Chloropren), wobei die Monomere hauptsächlich 1,4-verknüpft werden. Die Doppelbindungen in der Polymerkette des Chloropren-Kautschuks sind sowohl in *trans*-als auch in *cis*-Stellung zu finden, wobei das Verhältnis *trans*/*cis* etwa 9:1 beträgt. Die Chloratome sind sehr reaktionsträge und tragen zur Stabilität und Beständigkeit von Polychloropren bei. In sehr geringem Maße tritt auch ein Einbau von Monomeren in 1,2- oder 3,4-Stellung auf.

Das langfristige Ziel der Entwicklungsbemühungen ist es daher, einen Synthesekautschuk mit Hilfe von Enzymen *ex vivo* herzustellen mit verbesserten Eigenschaften entsprechend jenen des natürlichen Kautschuks.

Aufgabe der Erfindung

Die technische Aufgabe der vorliegenden Erfindung ist es, Derivate von Isoprendiphosphaten mit Hilfe von Enzymen zu polymerisieren bzw. entsprechende Polymere enzymatisch herzustellen.

Beschreibung der Erfindung

Die technische Aufgabe wird in einem ersten Aspekt gelöst durch ein Verfahren zur enzymatischen Verkettung von Monomeren enthaltend die Schritte:

a) Verketten von Monomeren mit der Formel (I)

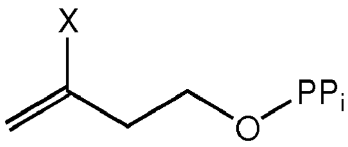

(I),

wobei X ein Halogenatom und O-$PP_i$ eine Diphosphatgruppe ist,
und optional von Isopentenyldiphosphat (IPP) als weiterem Monomer, wobei die Monomere mit einer Prenyltransferase und einem allylischen Diphosphat als Startersubstrat in Anwesenheit zweiwertiger Ionen in Kontakt gebracht werden;
b) Erhalten von Produkten mit Molekülketten, die Monomereinheiten enthalten, die durch die Formel (II) dargestellt werden:

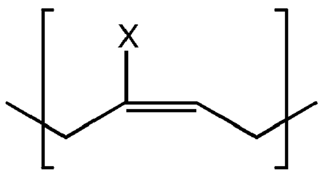

(II)

wobei X wie oben definiert ist, und
optional, wobei die Molekülketten zusätzlich Monomereinheiten enthalten, die durch die Formel (III) dargestellt werden:

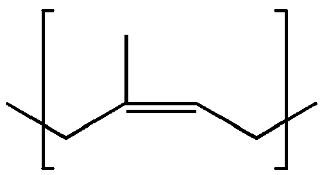

(III)

c) optional, Abspalten der Diphosphatgruppen von den in b) erhaltenen Produkten;
mit der Maßgabe, dass, wenn Isopentenyldiphosphat nicht als weiteres Monomer in der Reaktion eingesetzt wird

- und wenn X Brom ist, die Produkte mit Molekülketten mehr als 2 Monomereinheiten mit der Formel (II) aufweisen;
- und wenn X Chlor ist, die Produkte mit Molekülketten mehr als 1 Monomereinheit mit der Formel (II) aufweisen;

und
mit der Maßgabe, dass, wenn Isopentenyldiphosphat als weiteres Monomer in der Reaktion eingesetzt wird, der Anteil der Produkte mit Molekülketten, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht beträgt.

Isopentenyldiphosphat weist folgende Formel (IX) auf

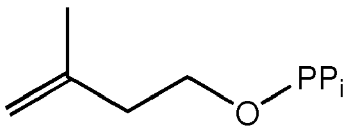

(IX).

Isopentenyldiphosphat wird auch Isopentenylpyrophosphat genannt oder abgekürzt mit "IPP" bezeichnet.

In einer Ausführungsform des erfindungsgemäßen Verfahrens werden Produkte mit Molekülketten erhalten, die Monomereinheiten enthalten, die durch die Formel (II) dargestellt werden:

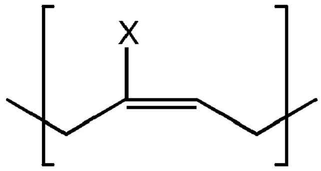

(II)

wobei X wie oben definiert ist, und wobei die Molekülketten zusätzlich Monomereinheiten enthalten, die durch die Formel (III) dargestellt werden:

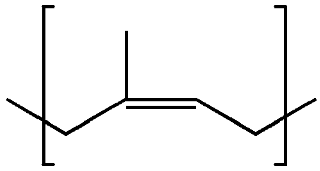

(III),

nämlich dann wenn Isopentenyldiphosphat in der Reaktionsmischung vorhanden ist.

In einer alternativen Ausführungsform des erfindungsgemäßen Verfahrens werden Produkte mit Molekülketten erhalten, die lediglich Monomereinheiten enthalten, die durch die Formel (II) dargestellt werden:

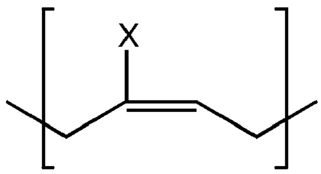

(II)

wobei X wie oben definiert ist, und die Molekülketten keine Monomereinheiten enthalten, die durch die Formel (III) dargestellt werden:

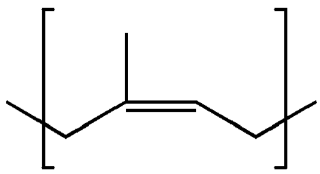

(III),

nämlich dann wenn Isopentenyldiphosphat in der Reaktionsmischung nicht enthaltend ist.

In einem zweiten Aspekt stellt die vorliegende Erfindung ein Verfahren bereit zur enzymatischen Herstellung von Produkten durch Umsetzen eines Reaktionsgemisches enthaltend allylisches Diphosphat als Startersubstrat und 3-Halo-3-Butenyl-1-Diphosphat als Monomer zur Elongation in Anwesenheit einer Prenyltransferase und zweiwertiger Ionen, wobei optional zusätzlich Isopentenyldiphosphat als weiteres Monomer zur Elongation in dem Reaktionsgemisch enthalten sein kann,
mit der Maßgabe, dass, wenn Isopentenyldiphosphat nicht als weiteres Monomer eingesetzt wird und

- wenn X Brom ist, die Produkte mit Molekülketten mehr als 2 Monomereinheiten mit der Formel (II) aufweisen;
- wenn X Chlor ist, die Produkte mit Molekülketten mehr als 1 Monomereinheit mit der Formel (II) aufweisen;

und

mit der Maßgabe, dass, wenn Isopentenyldiphosphat als weiteres Monomer in der Reaktion eingesetzt wird, der Anteil der Produkte, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht beträgt.

In bevorzugter Weise werden dabei Produkte erhalten, die im Elongationsbereich des gebildeten Moleküls, insbesondere des Polymermoleküls, mindestens eine 3-Halo-But-2-en-Einheit, und optional mindestens eine Isopren-Einheit enthalten.

Weiterhin stellt die vorliegende Erfindung in einem dritten Aspekt ein Verfahren bereit zur enzymatischen Herstellung von Produkten, enthaltend die Schritte:

a) Umsetzen eines Reaktionsgemisches enthaltend allylisches Diphosphat als Startermolekül und Monomere mit der Formel (I) als Elongationssubstrat

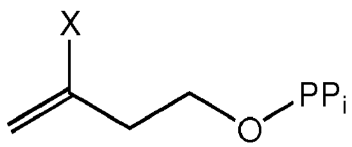

(I),

wobei X ein Halogenatom und O-$PP_i$ eine Diphosphatgruppe ist,
und optional von Isopentenyldiphosphat als weiterem Monomer in Anwesenheit einer Prenyltransferase und zweiwertiger Ionen;

b) Erhalten von Produkten mit Molekülketten, die Monomereinheiten enthalten, die durch die Formel (II) dargestellt werden:

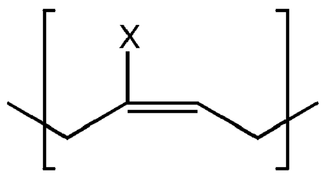

(II)

wobei X wie oben definiert ist, und
optional, wobei die Molekülketten zusätzlich Monomereinheiten enthalten, die durch die Formel (III) dargestellt werden:

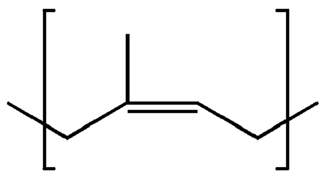

(III)

c) optional: Abspalten der Diphosphatgruppe von den in b) erhaltenen Produkten;
mit der Maßgabe, dass, wenn Isopentenyldiphosphat nicht als weiteres Monomer in der Reaktion eingesetzt wird und

- wenn X Brom ist, die Produkte mit Molekülketten mehr als 2 Monomereinheiten mit der Formel (II) aufweisen;
- wenn X Chlor ist, die Produkte mit Molekülketten mehr als 1 Monomereinheit mit der Formel (II) aufweisen;

und

mit der Maßgabe, dass, wenn Isopentenyldiphosphat als weiteres Monomer in der Reaktion eingesetzt wird, der Anteil der Produkte, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht beträgt.

Nachfolgend werden die bevorzugten Ausführungsformen zu dem ersten, zweiten und dritten Aspekt der vorliegenden Erfindung erläutert.

In einer bevorzugten Ausführungsform werden durch das erfindungsgemäße Verfahren Produkte mit der Formel (IV) erhalten

$$R^1\text{-}A_n\text{-}B_m\text{-}O\text{-}PP_i \qquad (IV),$$

wobei $R^1$ der Rest eines allylischen Startersubstrats ist, A die Monomereinheit $[CH_2\text{-}C(X)=CH\text{-}CH_2]$ und B die Monomereinheit $[CH_2\text{-}C(CH_3)=CH\text{-}CH_2]$ darstellt, wobei X ein Halogenatom, O-$PP_i$ eine Diphosphatgruppe oder durch O-H ersetzt ist, n eine ganze Zahl von 1 bis 30000 und m eine ganze Zahl von 0 bis 30000 ist, wobei die Summe von n und m eine ganze Zahl von 1 bis 30000 ist,
wobei, wenn m nicht 0 ist, die Monomereinheiten A und B in ihrer Anordnung statistisch verteilt sind oder A-O-$PP_i$ endständig angeordnet ist,
mit der Maßgabe, dass, wenn m = 0 ist, und

- wenn X ein Bromatom ist, n größer als 2 ist oder
- wenn X ein Chloratom ist, n größer als 1 ist.

Wenn m = 0 ist, dann werden die Monomereinheiten A in cis-Stellung miteinander verknüpft. Wenn m nicht 0 ist, dann werden die Monomereinheiten A und B in cis-Stellung verknüpft.

Der Substituent $R^1$ in der Formel (IV), der den Rest des allylischen Startersubstrats darstellt, weist bevorzugt die folgende allgemeine Formel (VIII) auf:

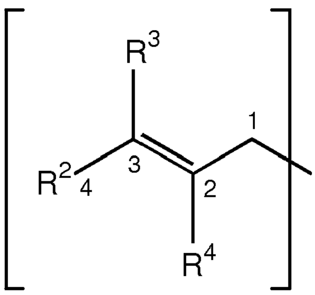
(VIII),

wobei $R^3$ Methyl oder Halogen, $R^4$ Halogen oder Wasserstoff ist und $R^2$ ausgewählt ist aus der Gruppe enthaltend vorzugsweise Prenyl-, Geranyl-, Farnesylgruppe, die weitere Substituenten bzw. funktionelle Gruppen aufweisen können.

Weiterhin ist bevorzugt, dass zusätzlich Isopentenyldiphosphat als weiteres Monomer bei der Reaktion anwesend ist. Damit stehen der Prenyltransferase bei der Polymerisierung mindestens zwei verschiedene Monomere zur Kettenverlängerung bereit, so dass im Elongationsbereich des von der Prenyltransferase erzeugten Moleküls, insbesondere eines Polymermoleküls, mindestens zwei verschiedene Monomere eingebaut werden, das als Copolymer bezeichnet werden kann.

In einem bevorzugten Verfahren werden daher Produkte mit Molekülketten erhalten, die in denselben Molekülketten Monomereinheiten der Formeln (II) und (III) enthalten:

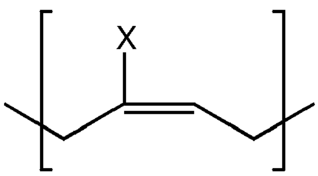
(II),

wobei X ein Halogenatom ist,
und

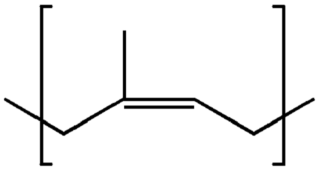

(III).

Ein einem alternativen bevorzugten Verfahren wird Isopentenyldiphosphat bei der Reaktion nicht verwendet und die Produkte enthalten Monomereinheiten, die durch die Formel (II) dargestellt sind:

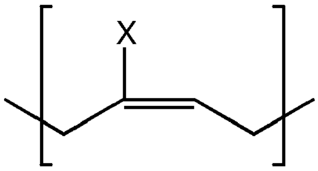

(II),

wobei X ein Halogenatom ist.

In einem weiteren bevorzugten Verfahren ist mindestens bei 0,1 Gew.-%, bevorzugt mindestens 1 Gew.-% bezogen auf das Trockengewicht der Produkte die Zahl der Monomereinheiten, die durch die Formel (II) dargestellt werden, eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Insbesondere ist in einem weiteren Verfahren bevorzugt, dass mindestens bei 0,1 Gew.-%, bevorzugt mindestens 1 Gew.-% bezogen auf das Trockengewicht der Produkte die Zahl der Monomereinheiten im Elongationsbereich eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, besonders bevorzugt 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

In einem alternativen bevorzugten Verfahren ist mindestens bei 0,1 Gew.-%, bevorzugt mindestens 1 Gew.-% bezogen auf das Trockengewicht der Produkte die Zahl der Monomereinheiten mit der Formel (II) und Formel (III) in der gleichen Molekülkette insgesamt eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8. Dabei besteht der genannte Anteil der Produkte aus Molekülketten, die jeweils mindestens eine Monomereinheit mit der Formel (II) und mindestens eine Monomereinheit mit der Formel (III) aufweisen.

Weiterhin ist bevorzugt, dass X ausgewählt ist aus der Gruppe bestehend aus Fluor, Chlor, Brom und Iod. Damit wird in bevorzugten Verfahren das Monomer ausgewählt aus der Gruppe bestehend aus 3-Brom-3-Butenyldiphosphat, 3-Chlor-3-Butenyldiphosphat, 3-Fluor-3-Butenyldiphosphat und 3-Iod-3-Butenyldiphosphat.

In einem weiteren bevorzugten Verfahren ist die Prenyltransferase (Prenyldiphosphatsynthase) eine *cis*-Prenyltransferase.

Insbesondere ist die Prenyltransferase in der Lage, bei Verwendung des Elongationssubstrates Isoprenyldiphosphat und Farnesyldiphosphat als Startersubstrat Produkte, insbesondere Polymere, mit einer Kettenlänge von mindestens $C_{20}$ zu synthetisieren, vorzugsweise mit einer Kettenlänge von $C_{20}$ bis $C_{150000}$, bevorzugt mit einer Kettenlänge von $C_{20}$ bis $C_{5000}$, weiter bevorzugt mit einer Kettenlänge von $C_{20}$ bis $C_{120}$, weiter bevorzugt mit einer Kettenlänge von $C_{20}$ bis $C_{90}$, besonders bevorzugt mit einer Kettenlänge von $C_{20}$ bis $C_{85}$. insbesondere bevorzugt mit einer Kettenlänge von $C_{20}$ bis $C_{65}$, und am meisten bevorzugt mit einer Kettenlänge von $C_{20}$ bis $C_{55}$.

Insbesondere wird die Prenyltransferase (Prenyldiphosphatsynthase) ausgewählt aus der Gruppe bestehend aus *E. coli* Undecaprenyldiphosphatsynthase (EcUPPS, EC: 2.5.1.31; SEQ ID NO: 1), *Thermococcus kodakaraensis cis*-Prenyltransferase (ThkCPT, EC: 2.5.1.87; SEQ ID NO: 2), *Micrococcus luteus* Undecaprenyldiphosphatsynthase (MlUPPS, EC: 2.5.1.31; SEQ ID NO: 3), *Mycobacterium tuberculosis* Undecaprenyldiphosphatsynthase (MtUPPS, EC: 2.5.1.86 und 2.5.1.87; SEQ ID NO: 4), *Helicobacter pylori* Undecaprenyldiphosphatsynthase (HpUPPS, EC: 2.5.1.31; SEQ ID NO: 5), menschliche Dehydrodolichyldiphosphat-Synthase (EC: 2.5.1.87; SEQ ID NO: 6), und Kautschuk-Synthasen, insbesondere bevorzugt aus *Hevea brasiliensis* (EC 2.5.1.20; SEQ ID NO: 7) und *Taraxacum koksaghyz* (EC 2.5.1.20; SEQ ID NO: 8), Dehydrodolichyldiphosphat-Synthase aus *Arabidopsis thaliana* (EC 2.5.1.87; SEQ ID NO: 9) eingesetzt.

Als weitere Prenyltransferasen, die für die vorliegende Erfindung verwendet werden können, sind Geranyldiphosphatsynthase, Farnesyldiphosphatsynthase, Geranylgeranyldiphosphatsynthase, Geranylfarnesyldiphosphatsynthase und Hexaprenyldiphosphatsynthase, Octaprenyldiphosphatsynthase usw. zu nennen.

Insbesondere wird ein Verfahren bevorzugt, wobei das allylische Diphosphat 1,1-Dimethylallyldiphosphat, Geranyldiphosphat, Farnesyldiphosphat, Geranylgeranyldiphosphat, Neryldiphosphat oder eine Verbindung ist, die eine allylische Diphosphatendgruppe oder ein funktionalisiertes allylisches Diphosphat enthält, insbesondere (2E,6E)-8-O-(*N*-methyl-2-aminobenzoyl)-3,7-dimethyl-2,6-octandien-1-diphosphat **(110).**

In einem weiteren bevorzugten Verfahren sind die zweiwertigen Ionen Metallionen, bevorzugt $Mg^{2+}$, $Mn^{2+}$ oder $Co^{2+}$ und besonders bevorzugt $Mg^{2+}$.

Weiterhin ist bevorzugt, dass das Verfahren bei einer Temperatur von 0°C bis 120 °C, bevorzugt von 0 °C bis 100 °C, weiter bevorzugt von 10 bis 70 °C, noch weiter bevorzugt von 20 bis 70 °C, und besonders bevorzugt im Bereich der optimalen Reaktionstemperatur der Enzyme durchgeführt wird.

Die vorliegende Erfindung stellt zudem Produkte mit der Formel (IV) bereit:

$$R^1\text{-}A_n\text{-}B_m\text{-O-PP}_i \qquad (IV),$$

wobei $R^1$ der Rest eines allylischen Startersubstrats ist, A die Monomereinheit $[CH_2\text{-}C(X)=CH\text{-}CH_2]$ und B die Monomereinheit $[CH_2\text{-}C(CH_3)=CH\text{-}CH_2]$ darstellt, wobei X ein Halogenatom, O-$PP_i$ eine Diphosphatgruppe oder durch O-H ersetzt ist, n eine ganze Zahl von 1 bis 30000 und m eine ganze Zahl von 0 bis 30000 ist, wobei die Summe von n und m eine ganze Zahl von 1 bis 30000 ist,
wobei, wenn m nicht 0 ist, die Monomereinheiten A und B in ihrer Anordnung statistisch verteilt sind oder A-O-$PP_i$ endständig angeordnet ist,
mit der Maßgabe, dass, wenn m = 0 ist, und

- wenn X ein Bromatom ist, n größer als 2 ist oder
- wenn X ein Chloratom ist, n größer als 1 ist.

Die Definition für $R^1$ wurde bereits oben erläutert.

Wenn m = 0 ist, dann sind die Monomereinheiten A in cis-Stellung miteinander verknüpft. Wenn m nicht 0 ist, dann sind die Monomereinheiten A und B in cis-Stellung verknüpft.

Insbesondere ist bevorzugt, dass m = 0 ist. In einer weiteren bevorzugten Ausführungsform des Produktes ist m = 0 und n ist eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Weiterhin stellt die vorliegende Erfindung Produkte mit der Formel (V) bereit:

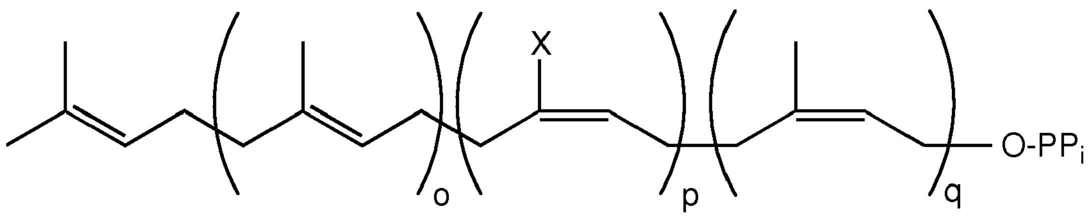

(V),

wobei X ein Halogenatom, O-$PP_i$ eine Diphosphatgruppe oder durch O-H ersetzt ist, o eine ganze Zahl von 1 bis 6, p eine ganze Zahl von 1 bis 30000 und q eine ganze Zahl von 0 bis 30000 ist, wobei die Summe von p und q eine ganze Zahl von 1 bis 30000 ist,
wobei, wenn q nicht 0 ist, die mit p und q bezeichneten Monomereinheiten in ihrer Anordnung statistisch verteilt sind oder die mit p bezeichnete Monomereinheit mit O-$PP_i$ endständig angeordnet ist,
mit der Maßgabe, dass, wenn q = 0 ist, und

- wenn X ein Bromatom ist, p größer als 2 ist oder
- wenn X ein Chloratom ist, p größer als 1 ist.

Bevorzugt ist o 1, 2 oder 3, insbesondere 2 oder 3 und besonders bevorzugt 2.

In einer weiteren bevorzugten Ausführungsform des Produktes ist q = 0 und p ist eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Die Erfindung stellt zudem Produkte mit der Formel (VI) bereit:

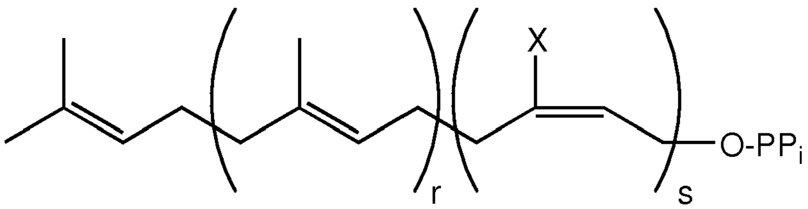

(VI),

wobei X ein Halogenatom, O-$PP_i$ eine Diphosphatgruppe oder durch O-H ersetzt ist, r eine ganze Zahl von 1 bis 6 und s eine ganze Zahl von 1 bis 30000 ist, mit der Maßgabe, dass,

- wenn X ein Bromatom ist, s größer als 2 ist oder
- wenn X ein Chloratom ist, s größer als 1 ist.

Bevorzugt ist r 1, 2 oder 3, insbesondere 2 oder 3 und besonders bevorzugt 2.

In einer weiteren bevorzugten Ausführungsform des Produktes ist s eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Die Erfindung stellt weiterhin die Verwendung von Prenyltransferasen zur Herstellung von Produkten mit Molekülketten bereit, die Monomereinheiten enthalten, die durch die Formel (II) dargestellt werden:

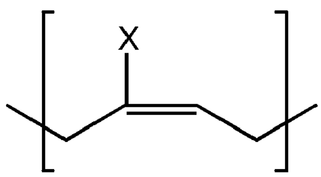

(II)

wobei X ein Halogenatom ist, und
optional, wobei die Molekülketten zusätzlich Monomereinheiten enthalten, die durch die Formel (III) dargestellt werden:

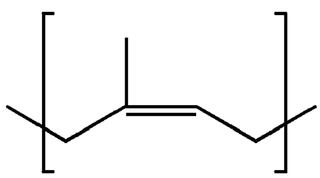

(III),

mit der Maßgabe, dass, wenn die Monomereinheiten mit der Formel (III) nicht enthalten sind

- und wenn X Brom ist, die Produkte mit Molekülketten mehr als 2 Monomereinheiten mit der Formel (II) aufweisen;
- und wenn X Chlor ist, die Produkte mit Molekülketten mehr als 1 Monomereinheit mit der Formel (II) aufweisen;

und
mit der Maßgabe, dass, wenn die Monomereinheiten mit der Formel (III) enthalten ist, der Anteil der Produkte mit Molekülketten, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht

beträgt.

In einer bevorzugten Verwendung werden Produkte mit Molekülketten erhalten, die in denselben Molekülketten Monomereinheiten der Formel (II) und (III) enthalten:

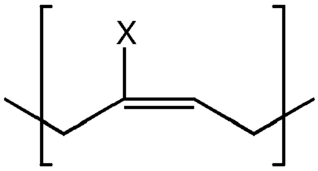
(II),

wobei X ein Halogenatom ist,
und

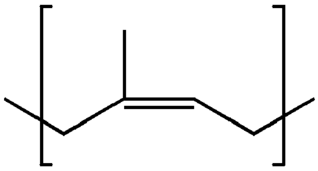
(III).

In einer weiteren bevorzugten Verwendung ist mindestens bei 0,1 Gew.-%, bevorzugt mindestens 1 Gew.-% bezogen auf das Trockengewicht der Produkte die Zahl der Monomereinheiten eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Die Erfindung stellt zudem die Verwendung von Prenyltransferasen zur Herstellung von Produkten mit der Formel (IV) bereit

$$R^1\text{-}A_n\text{-}B_m\text{-O-PP}_i \qquad \text{(IV)},$$

wobei $R^1$ der Rest eines allylischen Startersubstrats ist, A die Monomereinheit $[CH_2\text{-}C(X)\text{=}CH\text{-}CH_2]$ und B die Monomereinheit $[CH_2\text{-}C(CH_3)\text{=}CH\text{-}CH_2]$ darstellt, wobei X ein Halogenatom, O-$PP_i$ eine Diphosphatgruppe oder durch O-H ersetzt ist, n eine ganze Zahl von 1 bis 30000 und m eine ganze Zahl von 0 bis 30000 ist, wobei die Summe von n und m eine ganze Zahl von 1 bis 30000 ist,
wobei, wenn m nicht 0 ist, die Monomereinheiten A und B in ihrer Anordnung statistisch verteilt sind oder A-O-$PP_i$ endständig angeordnet ist
mit der Maßgabe, dass, wenn m = 0 ist, und

- wenn X ein Bromatom ist, n größer als 2 ist oder

- wenn X ein Chloratom ist, n größer als 1 ist.

Wenn m = 0 ist, dann werden die Monomereinheiten A in cis-Stellung miteinander verknüpft. Wenn m nicht 0 ist, dann werden die Monomereinheiten A und B in cis-Stellung verknüpft.

Dabei ist bevorzugt, dass m = 0 ist. In einer weiteren bevorzugten Verwendung ist m = 0 und n ist eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

Die vorliegende Erfindung stellt weiterhin die Verwendung von Prenyltransferasen zur Herstellung von Produkten mit der Formel (V) bereit:

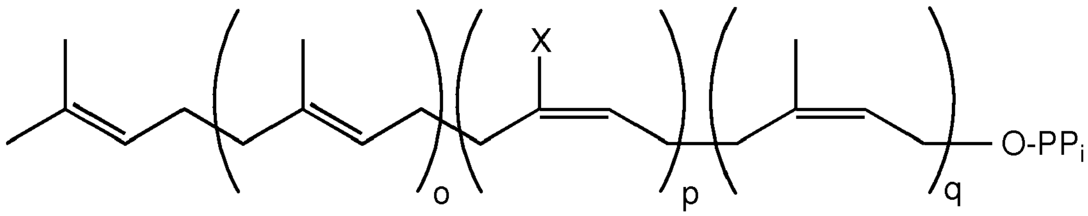

(V),

wobei X ein Halogenatom, O-$PP_i$ eine Diphosphatgruppe oder durch O-H ersetzt ist und o eine ganze Zahl von 1 bis 6, p eine ganze Zahl von 1 bis 30000 und q eine ganze Zahl von 0 bis 30000 ist, wobei die Summe von p und q eine ganze Zahl von 1 bis 30000 ist, und
wobei, wenn q nicht 0 ist, die mit p und q bezeichneten Monomereinheiten in ihrer Anordnung statistisch verteilt sind oder die mit p bezeichnete Monomereinheit zusammen mit O-$PP_i$ endständig angeordnet ist, mit der Maßgabe, dass, wenn q = 0 ist, und

- wenn X ein Bromatom ist, p größer als 2 ist oder
- wenn X ein Chloratom ist, p größer als 1 ist.

In einer weiteren bevorzugten Verwendung ist q = 0 und p ist eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.
Insbesondere stellt die Erfindung die Verwendung von Prenyltransferasen zur Herstellung von Produkten mit der Formel (VI) bereit:

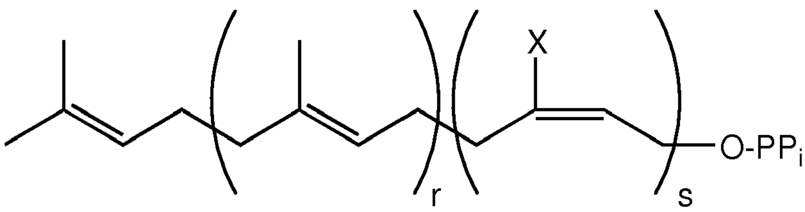

(VI),

wobei X ein Halogenatom, O-$PP_i$ eine Diphosphatgruppe oder durch O-H ersetzt ist, r eine ganze Zahl von 1 bis 6 und s eine ganze Zahl von 1 bis 30000 ist, mit der Maßgabe, dass,

- wenn X ein Bromatom ist, s größer als 2 ist oder
- wenn X ein Chloratom ist, s größer als 1 ist.

In einer weiteren bevorzugten Verwendung ist s eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8, insbesondere 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.
Bevorzugt ist X ausgewählt ist aus der Gruppe bestehend aus Fluor, Chlor, Brom und Iod. Weiter bevorzugt ist die die Prenyltransferase eine *cis*-Prenyltransferase ist. Insbesondere ist die Prenyltransferase eine Prenyldiphosphatsynthase ist, die dadurch gekennzeichnet ist, dass diese bei Verwendung des Elongationssubstrates Isoprenyldiphosphat und Farnesyldiphosphat als Startersubstrat Produkte mit einer Kettenlänge von mindestens $C_{20}$ synthetisieren, vorzugsweise mit einer Kettenlänge von $C_{20}$ bis $C_{150000}$, bevorzugt mit einer Kettenlänge von $C_{20}$ bis $C_{5000}$, weiter bevorzugt mit einer Kettenlänge von $C_{20}$ bis $C_{120}$, weiter bevorzugt mit einer Kettenlänge von $C_{20}$ bis $C_{90}$, besonders bevorzugt mit einer Kettenlänge von $C_{20}$ bis $C_{85}$. insbesondere bevorzugt mit einer Kettenlänge von $C_{20}$ bis $C_{65}$, und am meisten bevorzugt mit einer Kettenlänge von $C_{20}$ bis $C_{55}$.
Noch weiter bevorzugt ist die Prenyltransferase ausgewählt aus der Gruppe bestehend aus *E. coli* Undecaprenyldiphosphatsynthase (EcUPPS, EC: 2.5.1.31; SEQ ID NO: 1), *Thermococcus kodakaraensis cis*-Prenyltransferase (ThkCPT, EC: 2.5.1.87; SEQ ID NO: 2), *Micrococcus luteus* Undecaprenyldiphosphatsynthase (MIUPPS, EC: 2.5.1.31;

SEQ ID NO: 3), *Mycobacterium tuberculosis* Undecaprenyldiphosphatsynthase (MtUPPS, EC: 2.5.1.86 und 2.5.1.87; SEQ ID NO: 4), *Helicobacter pylori* Undecaprenyldiphosphatsynthase (HpUPPS, EC: 2.5.1.31; SEQ ID NO: 5), menschliche Dehydrodolichyldiphosphat-Synthase (EC: 2.5.1.87; SEQ ID NO: 6), und Kautschuk-Synthasen, insbesondere bevorzugt aus *Hevea brasiliensis* (EC 2.5.1.20; SEQ ID NO: 7) und *Taraxacum koksaghyz* (EC 2.5.1.20; SEQ ID NO: 8), und Dehydrodolichyldiphosphat-Synthase aus *Arabidopsis thaliana* (EC 2.5.1.87; SEQ ID NO: 9).

**Beschreibung der Abbildungen**

**Abbildung 1** zeigt Fotographien von mit Coomassie®-Brilliant Blau gefärbten 14%igen Gelen einer SDS-PAGE mit Proben aus der EcUPPS-Proteinreinigung (A) bzw. aus der ThkCPT-Proteinreinigung (B). A: 1: Bakterienlysat vor der Induktion mit IPTG; 2: Bakterienlysat nach der Induktion mit IPTG; 3: unlösliche Bestandteile; 4: Bakterienlysat mit löslichen Proteinen; 5: Proteine, die nicht an die Metallaffinitätssäule gebunden haben; 6: Eluat der Affinitätschromatographie mit gereinigtem Protein; 7: entsalzte Proteinfraktion nach PD10-Säule; M: Marker.

**Abbildung 2** zeigt Fotographien von mit Coomassie®-Brilliant Blau gefärbten 14%igen Gelen einer SDS-PAGE mit Proben aus der Proteinreinigung der UPPS aus *Micrococcus luteus* (A), *Mycobacterium tuberculosis* (B) und *Helicobacter pylori* (C); (A): MIUPPS; M: Proteingrößenstandard (10-130 kDa); 1: Rohextrakt nach Induktion mit IPTG; 2: Protein nach Metall-Affinitätschromatographie-Reinigung und Entsalzung. (B) MtUPPS: M: Proteingrößenstandard (10-130 kDa); 1: Rohextrakt nach Induktion mit IPTG; 2: Rohextrakt ohne Prenyltransferase MtUPPS nach Auftragen über die $Ni^{2+}$-Säule; 3: Protein nach Metall-Affinitätschromatographie-Reinigung und Entsalzung; (C) UPPS aus *Helicobacter pylori.* M Proteingrößenstandard (10-130 kDa); 1: Rohextrakt vor Induktion mit IPTG; 2: Rohextrakt nach Induktion und Inkubation mit IPTG; 3: Protein nach Metall-Affinitätschromatographie-Reinigung und Entsalzung.

**Abbildung 3** zeigt die Aktivitäten der verschiedenen *cis*-Prenyltransferasen hinsichtlich der Umsetzung von FPP mit IPP. Als Negativkontrolle diente ein Ansatz ohne Enzym (nicht dargestellt). Es wurden folgende cis-Prenyltransferasen verwendet: Undecaprenyldiphosphatsynthase (UPPS) aus *Escherichia coli* (EcUPPS), aus *Micrococcus luteus* (MIUPPS), UPPS aus *Mycobacterium tuberculosis* (MtUPPS) und UPPS aus *Helicobacter pylori* (HpUPPS).

**Abbildung 4** zeigt die Aktivitäten der verschiedenen *cis*-Prenyltransferasen hinsichtlich der Umsetzung von FPP mit Cl-BPP. Als Negativkontrolle diente ein Ansatz ohne Enzym (nicht dargestellt). Folgende cis-Prenyltransferasen wurden verwendet: Undecaprenyldiphosphatsynthase (UPPS) aus *Escherichia coli* (EcUPPS), aus *Micrococcus luteus* (MIUPPS), UPPS aus *Mycobacterium tuberculosis* (MtUPPS) und UPPS aus *Helicobacterpylori* (HpUPPS).

**Abbildung 5** zeigt die artifiziellen Startersubstrate der EcUPPS von oben nach unten abnehmend geordnet nach ihrer relativen katalytischen Effizienz der EcUPPS mit dem jeweiligen Startersubstrat in Bezug zu dem natürlichen Substrat FPP.

**Abbildung 6** zeigt die getesteten artifiziellen Substrate, die von der EcUPPS nicht akzeptiert werden.

**Abbildung 7** zeigt ein HPL-Chromatogramm der Produkte der ThkCPT mit **110** und den Elongationssubstraten Br-BPP (gestrichelte Linie) und Cl-BPP (durchgezogene Linie). Die Detektion erfolgte über Fluoreszenz bei Ex 352 nm/Em 420 nm. Die Inkubationstemperatur betrug 25°C.

**Abbildung 8** zeigt ein HPL-Chromatogramm der Produkte der ThkCPT mit **110** und den Elongationssubstraten Br-BPP (gestrichelte Linie) und Cl-BPP(durchgezogene Linie). Die Detektion erfolgte über Fluoreszenz bei Ex 352 nm/Em 420 nm. Die Inkubationstemperatur betrug 65°C.

**Abbildung 9** zeigt HPL-Chromatogramme der Produkte der ThkCPT mit **110** und den Elongationssubstraten IPP (obere Abbildung), Cl-BPPim Gemisch mit IPP im Verhältnis 1:10 (mittlere Abbildung) und Br-BPP im Gemisch mit IPP im Verhältnis 1:10. Die Detektion erfolgte über Fluoreszenz bei Ex 352 nm/Em 420 nm.

**Detaillierte Beschreibung der Erfindung**

Die vorliegende Erfindung betrifft Verfahren gemäß dem ersten, zweiten und dritten Aspekt.

Insbesondere betrifft die vorliegende Erfindung ein Verfahren zur enzymatischen Verkettung von Monomeren

(erster Aspekt), enthaltend die Schritte:

a) Verketten von Monomeren mit der Formel (I)

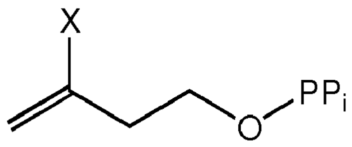

(I),

wobei X ein Halogenatom und O-$PP_i$ eine Diphosphatgruppe ist,
und optional von Isopentenyldiphosphat als weiterem Monomer,
wobei die Monomere mit einer Prenyltransferase und einem allylischen Diphosphat als Startersubstrat in Anwesenheit zweiwertiger Ionen in Kontakt gebracht werden;

b) Erhalten von Produkten mit Molekülketten, die Monomereinheiten enthalten, die durch die Formeln (II) dargestellt werden:

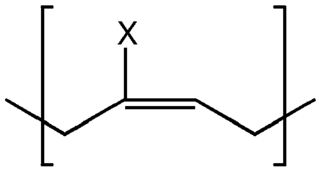

(II),

wobei X wie oben definiert ist, und
optional, wobei die Molekülketten zusätzlich Monomereinheiten enthalten, die durch die Formel (III) dargestellt werden:

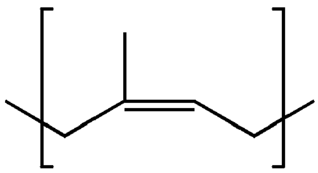

(III);

c) optional: Abspalten der Diphosphatgruppe von den in b) erhaltenen Produkten.

In einem zweiten Aspekt stellt die vorliegende Erfindung ein Verfahren zur enzymatischen Herstellung von Produkten, insbesondere von Polymeren, bereit, durch Umsetzen eines Reaktionsgemisches enthaltend allylisches Diphosphat als Startersubstrat und 3-Halo-3-Butenyl-1-Diphosphat als Monomer zur Elongation, in Anwesenheit einer Prenyltransferase und zweiwertiger Ionen, wobei optional zusätzlich Isopentenyldiphosphat als weiteres Monomer zur Elongation in dem Reaktionsgemisch enthalten sein kann.

In einem dritten Aspekt stellt die vorliegende Erfindung ein Verfahren bereit zur enzymatischen Herstellung von Produkten, insbesondere von Polymeren, enthaltend die Schritte:

a) Umsetzen eines Reaktionsgemisches enthaltend allylisches Diphosphat als Startermolekül und Monomere mit der Formel (I) als Elongationssubstrat

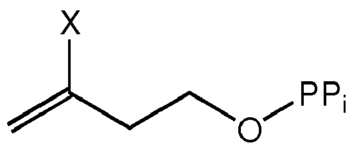

(I),

wobei X ein Halogenatom und O-$PP_i$ eine Diphosphatgruppe ist, und optional von Isopentenyldiphosphat als weiterem Monomer in Anwesenheit einer Prenyltransferase und zweiwertiger Ionen;

b) Erhalten von Produkten mit Molekülketten, die Monomereinheiten enthalten, die durch die Formel (II) dargestellt werden:

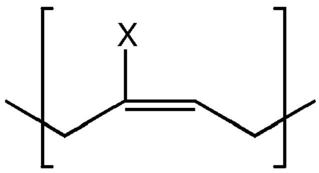
(II),

wobei X wie oben definiert ist, und
optional, wobei die Molekülketten zusätzlich Monomereinheiten enthalten, die durch die Formel (III) dargestellt werden:

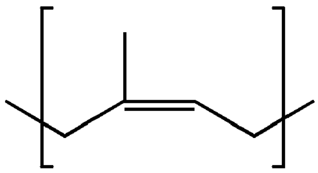
(III);

c) optional: Abspalten der Diphosphatgruppe von den in b) erhaltenen Produkten.

**Definitionen**

Bei den durch die erfindungsgemäßen Verfahren hergestellten Produkten handelt es sich um Substanzen mit linearen Molekülketten. Je nach Länge der Ketten, d.h. je nach der Anzahl der in den Ketten enthaltenen Monomereinheiten spricht man in der Regel von Oligomeren, Multimeren oder Polymeren. Die Produkte gemäß der vorliegenden Erfindung, also Oligomere, Multimere und Polymere, liegen in der Regel als Gemisch von Molekülen mit unterschiedlichen Kettenlängen vor. Allerdings kann ein Teil oder gar der Großteil der Produkte gerade aufgrund ihrer enzymatischen Herstellung aus einer oder wenigen Molekülspezies bzw. Molkülketten mit jeweils gleicher Kettenlänge bestehen. Die Produkte können dabei in wässriger Lösung oder in einem organischem Lösungsmittel verdünnt vorliegen, aufkonzentriert sein oder sie werden als Feststoff bereitgestellt.

Die gemäß der Verfahren der vorliegenden Erfindung hergestellten bzw. bereitgestellten Produkte werden im Rahmen dieser Anmeldung auch als "Produkte mit Molekülketten" bezeichnet. Dabei handelt es sich um lineare Molekülketten, mit einer 1,4-Verknüpfung der Monomereinheiten, insbesondere in cis-Stellung. Im Rahmen dieser Anmeldung können die gemäß der Verfahren der vorliegenden Erfindung hergestellten bzw. bereitgestellten Produkte auch als Polymere bezeichnet werden. Als Polymer im Sinne der vorliegenden Erfindung wird ein Stoff verstanden, der aus Molekülen besteht, die durch eine Sequenz einer oder mehrerer Arten von Monomereinheiten gekennzeichnet sind. Diese Moleküle liegen innerhalb eines bestimmten Molekulargewichtsbereichs, wobei die Unterschiede beim Molekulargewicht im Wesentlichen auf die Unterschiede in der Zahl der Monomereinheiten zurückzuführen sind. Ein Polymer enthält Polymermoleküle, wobei ein Polymermolekül definiert ist als ein Molekül, das eine Folge von mindestens drei Monomereinheiten enthält, die zumindest mit einer weiteren Monomereinheit bzw. einem sonstigen Reaktionsmittel eine kovalente Bindung eingegangen sind. Im Rahmen dieser Definition ist unter einer "Monomereinheit" die gebundene Form eines Monomers in einem Polymer zu verstehen. Eine "Sequenz" ist eine durchgehende Folge von Monomereinheiten innerhalb des Moleküls, die kovalent miteinander verbunden sind und von anderen Einheiten als den Monomereinheiten nicht unterbrochen werden. Der Begriff "sonstiges Reaktionsmittel" bezieht sich auf ein Molekül, das mit einem oder mehreren Sequenzen von Monomereinheiten verbunden werden kann, das aber unter den relevanten Reaktionsbedingungen, die für die Polymerbildung verwendet werden, nicht als Monomer angesehen werden kann.

Insbesondere wird als Polymer im Sinne der vorliegenden Erfindung eine Substanz verstanden, deren Struktur durch die allgemeine Formel -R-R-R- dargestellt werden kann, wobei "-R-" zweiwertige Radikale sind, die im Allgemeinen als solche nicht unabhängig existieren können. Bei den Polymeren gemäß der Erfindung handelt es sich insbesondere um Polymere, die durch eine Additionsreaktion oder Anlagerungsreaktion bereitgestellt werden können. Andererseits, diese Polymermoleküle können durch Hitzeeinwirkung in ein Monomer mit der gleichen Zusammensetzung wie die Struktureinheit -R- der Molekülkette umgewandelt werden (W.H. Carothers, J. Am. Chem. Soc., 51, 2548-2559 (1929)).

Die bei der vorliegenden Erfindung verwendeten Prenyltransferasen benötigen zur Bildung eines Polymermoleküls ein Startersubstrat und ein oder mehrere Monomermoleküle. Das erste Monomer wird an das jeweilige Startersubstrat kovalent gebunden und die weiteren Monomere an das jeweils zuletzt angebundene Monomer angefügt. Der

Begriff "Monomereinheit" bezieht sich auf den Teil des Moleküls, der ausgehend von dem Startersubstrat durch die Prenyltransferase an das Startersubstrat kovalent angefügt wird. Dieser Bereich des Moleküls wird mit dem Begriff "Elongationsbereich" bezeichnet. Der Elongationsbereich erstreckt sich nicht auf den Teil des Polymermoleküls, der sich vom Startersubstrat ableitet. Die in dieser Anmeldung genannte Zahl an in dem Polymer bzw. in dem Polymermolekül bzw. in dem Produkt mit Molekülketten vorhandenen Monomereinheiten bezieht sich auf den Elongationsbereich. Gemäß der Erfindung enthält der Elongationsbereich des Polymers bzw. des Polymermoleküls bzw. des Produktes mit Molekülketten Monomereinheiten, die durch die Formel (II) dargestellt werden:

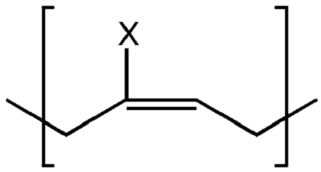
(II)

wobei X ein Halogenatom ist, und optional Monomereinheiten, die durch die Formel (III) dargestellt werden:

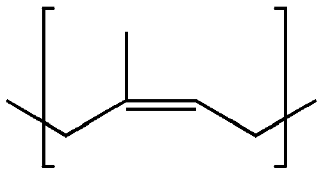
(III).

In einem bevorzugten Verfahren bzw. einer bevorzugten Ausführungsform des Polymers bzw. des Polymermoleküls bzw. des Produktes mit Molekülketten liegen die Monomereinheiten im Elongationsbereich in 1,4-Verknüpfung vor. In einem weiteren bevorzugten Verfahren bzw. einer bevorzugten Ausführungsform liegen die Monomereinheiten im Elongationsbereich in *cis*-Konfiguration vor.

Bei allen drei von der Erfindung zur Verfügung gestellten Verfahren ist bevorzugt, dass mindestens bei 0,1 Gew.-%, bevorzugt mindestens 1 Gew.-% bezogen auf die Trockensubstanz des Produktes oder Polymers die Zahl der Monomereinheiten eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8 ist. Weiter bevorzugt ist mindestens bei 0,1 Gew.-%, bevorzugt mindestens 1 Gew.-% bezogen auf die Trockensubstanz des Produktes oder Polymers die Zahl der Monomereinheiten eine ganze Zahl von 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8.

## Effekte und Vorteile der vorliegenden Erfindung

Die vorliegende Erfindung befasst sich insbesondere mit artifiziellen Verlängerungssubstraten und deren Einbau in Polymere durch entsprechende Prenyltransferasen. Bei den im Rahmen der vorliegenden Erfindung durchgeführten Versuche wurden eine Reihe bekannter *cis*-Prenyltransferasen (CPT) aus dem Bereich der Mittel- und Langketten-cis-Prenyltransferasen über Gensynthese und optimierte Expression in *E. coli* biosynthetisiert. Die Expression löslicher Enzyme ist bisher hauptsächlich bei den Mittelketten-cis-Prenyltransferasen gelungen. So konnte die *E. coli* Undecaprenyldiphosphatsynthase (EcUPPS) hinsichtlich ihres Starter- und Elongationssubstratspektrums gut charakterisiert werden. Zudem ist es gelungen die *cis*-Prenyltransferasen von *Thermococcus kodakaraensis* (ThkCPT), *Mycobacterium tuberculosis, Micrococcus luteus* und *Helicobacter pylori* zu reinigen und das Elongationssubstratspektrum mit Hilfe des MTP-Diphosphatassays zu bestimmen. Die Produkte, die von den Enzymen erzeugt werden konnten, wurden anschließend über DC, HPLC und LC/ESI-MS näher charakterisiert.

Überraschenderweise wurde dabei gefunden, dass als Elongationssubstrate IPP-Derivate als Substrat von den Enzymen akzeptiert und in das wachsende Molekül eingebaut wurden, wobei die Methylgruppe des Isopentenyldiphosphats an der Position C3 durch ein Halogen substituiert ist. Insbesondere wurden die artifiziellen Substrate 3-Brom-Butenyldiphosphat (Br-BPP) und 3-Chlor-Butenyldiphosphat (Cl-BPP) erfolgreich eingesetzt. Die Untersuchungen gemäß der vorliegenden Erfindung zeigen, dass alle untersuchten Enzyme 3-halogenierte Isopentenyldiphosphatderivate (z.B. Cl-BPP) als Substrat verwenden können. Die Enzyme können mit den 3-halogenierten Isopentenyldiphosphatderivaten Homopolymerketten wie auch zusammen mit dem natürlichen Substrat Isopentenyldisphophat Copolymer-Ketten erzeugen.

In dem Verfahren kann jede Prenyltransferase eingesetzt werden, die ein allylisches Diphosphat als Startersubstrat und ein Monomer gemäß der Formel (I) und optional Isopentenyldiphosphat als weiteres Monomer zur Elongation des Startersubstrats akzeptiert. Dabei kann die Prenyltransferase in aufgereinigter Form vorliegen, oder in Form eines

Mikroorganismus oder eines Zellextraktes desselben, der diese enzymatische Aktivität einer Prenyltransferase aufweist. Bei der Prenyltransferase kann es sich um ein natürlich vorkommendes Enzym, um eine verbesserte Mutante, um ein katalytisch aktives Fragment der Prenyltransferase oder um ein Fusionsprotein handeln, das mindestens ein katalytisch aktives Fragment der Prenyltransferase aufweist. Die Prenyltransferase kann auch an einen Träger gebunden, d. h. immobilisiert sein. Dabei wird der Träger mit der an diesem gebundenen Prenyltransferase zur Durchführung der Reaktion in der Reaktionsmischung verteilt oder die Reaktionsmischung wird zu diesem Zweck an dem Träger vorbeigeleitet.

Die Prenyltransferase, auch Polyprenylsynthase oder Prenyldiphosphatsynthase genannt, ist ein Enzym, das die fortlaufende 1-4-Verknüpfung von Isopentenyldiphosphat mit allylischen Diphosphaten als Startersubstrat katalysiert, wobei Polyprenyldiphosphate unterschiedlicher Kettenlänge entstehen. Die gemäß der Erfindung verwendete Prenyltransferase schließt damit alle Enzyme ein, das die Reaktion des kovalenten Verknüpfens einer oder mehrere Isopreneinheiten katalysiert.

In einem weiteren bevorzugten Verfahren ist die Prenyltransferase (Prenyldiphosphatsynthase) eine *cis*-Prenyltransferase.

Insbesondere ist die Prenyltransferase in der Lage bei Verwendung des Elongationssubstrates Isoprenyldiphosphat und Farnesyldiphosphat als Startersubstrat ein Polymer mit einer Kettenlänge von mindestens $C_{20}$ zu synthetisieren, vorzugsweise mit einer Kettenlänge von $C_{20}$ bis $C_{150000}$, bevorzugt mit einer Kettenlänge von $C_{20}$ bis $C_{5000}$, weiter bevorzugt mit einer Kettenlänge von $C_{20}$ bis $C_{120}$, weiter bevorzugt mit einer Kettenlänge von $C_{20}$ bis $C_{90}$, besonders bevorzugt mit einer Kettenlänge von $C_{20}$ bis $C_{85}$, insbesondere bevorzugt mit einer Kettenlänge von $C_{20}$ bis $C_{65}$ und am meisten bevorzugt mit einer Kettenlänge von $C_{20}$ bis $C_{55}$.

Weiterhin ist die Prenyltransferase (Prenyldiphosphatsynthase) vorzugsweise ausgewählt aus der Gruppe bestehend aus *E. coli* Undecaprenyldiphosphatsynthase (EcUPPS, EC: 2.5.1.31; SEQ ID NO: 1), *Thermococcus kodakaraensis cis*-Prenyltransferase (ThkCPT, EC: 2.5.1.87; SEQ ID NO: 2), *Micrococcus luteus* Undecaprenyldiphosphatsynthase (MlUPPS, EC: 2.5.1.31; SEQ ID NO: 3), *Mycobacterium tuberculosis* Undecaprenyldiphosphatsynthase (MtUPPS, EC: 2.5.1.86 und 2.5.1.87; SEQ ID NO: 4), *Helicobacterpylori* Undecaprenyldiphosphatsynthase (HpUPPS, EC: 2.5.1.31; SEQ ID NO: 5), menschliche Dehydrodolichyldiphosphat-Synthase (EC: 2.5.1.87; SEQ ID NO: 6), und Kautschuk-Synthasen, insbesondere bevorzugt aus *Hevea brasiliensis* (EC 2.5.1.20; SEQ ID NO: 7) und *Taraxacum koksaghyz* (EC 2.5.1.20; SEQ ID NO: 8), sowie Dehydrodolichyldiphosphat-Synthase aus *Arabidopsis thaliana* (EC 2.5.1.87; SEQ ID NO: 9).

Als weitere Prenyltransferasen, die für die vorliegende Erfindung verwendet werden können, sind Geranyldiphosphatsynthase, Farnesyldiphosphatsynthase, Geranylgeranyldiphosphatsynthase, Geranylfarnesyldiphosphatsynthase und Hexaprenyldiphosphatsynthase, Octaprenyldiphosphatsynthase usw. zu nennen.

Die Einteilung der Prenyltransferasen, die Polyprenyldiphosphate synthetisieren, erfolgt grundsätzlich nach den synthetisierten Produkten, in *cis*- und *trans*-Prenyltransferasen. Dabei unterscheidet man je nach Kettenlänge des entstehenden Produktes zwischen Kurz- (bis $C_{15}$), Mittel- (bis $C_{55}$) und Langketten-Prenyltransferasen (> $C_{55}$).

Cis-Prenyltransferasen können aus zwei kurzkettigen Isoprendiphosphaten hochmolekulare *cis*-Polyisoprene synthetisieren. Diese Enzyme verwenden als Startersubstrat Geranyl- (GPP), Farnesyl- (FPP) oder Geranylgeranyldiphosphat (GGPP) und katalysieren die Kettenverlängerung mit repetitiven Kondensationen des Elongationssubstrates Isopentenyldiphosphat (IPP). Die Startereinheiten werden von *trans*-Prenyltransferasen synthetisiert, die die Synthese von *trans*-Isoprendiphosphaten aus Dimethylallyldiphosphat (DMAPP) und IPP durchführen.

Bei den Mittelketten-cis-Prenyltransferasen handelt sich um lösliche Enzyme, die an der Synthese von Peptidoglykanen der bakteriellen Zellwand beteiligt sind. Als Lipidcarrier sind Polyprenyldiphosphate damit wesentlich an dem Aufbau der Zellwand beteiligt. Manche Enzyme weisen eine Restriktion der Produktkettenlänge auf, die auf Aminosäurereste zurückzuführen sind, die eine Art Deckel bilden und somit zum Kettenabbruch der wachsenden Produkte führen. Varianten ohne Deckel sind ebenfalls zugänglich.

Grundsätzlich benutzen *cis*-Prenyltransferasen stets ein allylisches Startersubstrat (z.B. Dimethylallyl- (DMAPP), Geranyl- (GPP), Farnesyl- (FPP), Geranylgeranyldiphosphat (GGPP)) sowie Isopentenyldiphosphat (IPP) als Verlängerungseinheit. Das Startersubstrat ist ein allylisches Diphosphat, d.h. eine Verbindung, die eine Allyl-Diphosphatgruppe enthält. Insbesondere kann als Startersubstrat eine Verbindung verwendet werden, mit einer 2-Butenylgruppe, die eine Diphosphatgruppe am $C_1$-Atom, eine Doppelbindung zwischen dem $C_2$-Atom und dem $C_3$-Atom und eine Methylgruppe oder eine Ethylgruppe am $C_3$-Atom enthält.

Für die Startersubstrate konnten einige Kriterien gezeigt werden, die für die Katalyse notwendig sind, wobei einige der im Folgenden beschriebenen Beobachtungen bereits im Stand der Technik beschrieben wurden und in den im Rahmen der vorliegenden Erfindung gemachten Versuche bestätigt werden konnten. Die folgende Formel (VII) stellt die allgemeine Formel für das Startersubstrat dar:

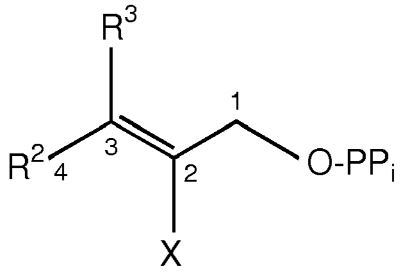

(VII).

Die Allyl-Diphosphatgruppe ist ein entscheidendes Merkmal. Wird ein Prenylmonophosphat als Startersubstrat oder ein Prenylbisphosphonat verwendet, kann keine Reaktion stattfinden. Auch eine Thioesterbindung zwischen Phosphat und Prenylrest verhindert die Katalyse zum Polyprenylprodukt. Ein weiteres wichtiges Kriterium ist die Methylgruppe in 3-Stellung des Allyl-Diphosphates ($R^3$ = Methylrest). Im Stand der Technik wurde anhand von Kurzketten-Prenyltransferasen gezeigt, dass $R^3$ auch durch ein Halogenatom dargestellt werden kann. Anhand der *trans*-Prenyltransferasen Hexa- und Heptaprenyldiphosphatsynthase wurde gezeigt, dass eine C3-Methylverzweigung des allylischen Substrates essentiell für die Kettenverlängerung ist. Eine zusätzliche Methylgruppe an C4 des Startersubstrates kann von der *Bacillus subtilis* UPPS, je nach Konfiguration, nicht oder nur mit sehr schlechten Geschwindigkeiten umgesetzt werden. Dagegen kann ein an C2 halogeniertes Startermolekül (X = Halogen) durchaus als Substrat dienen. Veränderungen am Startersubstrat, die räumlich weiter entfernt sind von der Diphosphatgruppe, haben wenig Einfluss auf die Substraterkennung und werden gut akzeptiert.

Insbesondere wird ein Verfahren bevorzugt, wobei das allylische Diphosphat 1,1-Dimethylallyldiphosphat, Geranyldiphosphat, Farnesyldiphosphat, Geranylgeranyldiphosphat, Neryldiphosphat oder eine Verbindung ist, die eine allylische Diphosphatendgruppe oder ein funktionalisiertes allylisches Diphosphat enthält, insbesondere (2E,6E)-8-O-(*N*-methyl-2-aminobenzoyl)-3,7-dimethyl-2,6-octandien-1-diphosphat **(110).**

Wie bereits erläutert, benutzen *cis*-Prenyltransferasen stets ein allylisches Startersubstrat (Dimethylallyl- (DMAPP), Geranyl- (GPP), Farnesyl- (FPP), Geranylgeranyldiphosphat (GGPP)) sowie Isopentenyldiphosphat (IPP) als Verlängerungseinheit. Die *E. coli* Undecaprenyldiphosphatsynthase (EcUPPS) benutzt FPP als Startersubstrat welches durch achtfache Verlängerung mit IPP das $C_{55}$-Produkt Undecaprenyldiphosphat synthetisiert. Der Mechanismus der Kettenverlängerung ist teilweise verstanden aber im Detail noch unklar. Anhand der vorhandenen Kristallstrukturen konnten im Stand der Technik Aussagen über die Substratbindungsstellen gemacht werden. Allerdings ist die Katalyse mit einer enormen strukturellen Veränderung des Enzyms verbunden, so dass die aktive Tasche an einer sehr flexiblen Loop-Struktur stets schlecht aufgelöst ist. Dazu kommt, dass das Enzym während der Katalyse von einer offenen in eine geschlossene Form wechselt, so dass die Lage der Aminosäurereste während der Synthese in einer starren Kristallstruktur nur schlecht abzuleiten ist.

Grundsätzlich ist die Katalyse wie folgt verstanden: IPP bindet magnesiumabhängig, während FPP Magnesiumunabhängig binden kann. Anschließend wird das Magnesiumion zum Diphosphat des FPP transferiert wo es die Abspaltung des Diphosphates begünstigt. Gleichzeitig greift die elektronenreiche Doppelbindung von IPP an dem dissoziierenden FPP an. Die katalytische Base abstrahiert ein Proton von IPP und führt damit zur Bildung einer neuen cis-Doppelbindung. Anschließend wird das Produkt von der IPP-Bindungsstelle zur FPP-Bindungstasche transferiert, um die erneute Bindung von IPP zu ermöglichen und einen weiteren Elongationsschritt zu erlauben. Die Reaktion verbraucht (mind.) ein Magnesiumion pro Zyklus.

Die vorliegende Erfindung wird beispielhaft anhand der folgenden Beispiele erläutert, die aber keinesfalls als Einschränkung der Erfindung oder des Schutzbereiches verstanden werden sollen.

## Beispiele

### Beispiel 1: Proteinexpression, Zellaufschluss und Reinigung zur Gewinnung der Prenyltransferasen EcUPPS und ThkCPT

1.1 Expression

Zur Expression der Prenyltransferasen *Escherichia coli* Undecaprenyldiphosphatsynthasen und *Thermococcus kodakaraensis cis*-Prenyltransferase wurde eine Vorkultur in einem sterilen Erlenmeyerkolben in LB-Medium mit dem entsprechenden Antibiotikum aus einer Glycerinkultur über Nacht bei 37°C und 200 UpM angezogen. Das Medium enthielt 100 $\mu$g-mL$^{-1}$ Ampicillin und 50 $\mu$g-mL$^{-1}$ Chloramphenicol im Fall der EcUPPS oder 50 $\mu$g-mL$^{-1}$ Kanamycin für die Expression der ThkCPT. Für die 500 mL Hauptkultur wurde die Vorkultur 1:20 (v/v) mit Antibiotikum haltigem Medium

verdünnt und bis zu einer $OD_{600}$ (optische Dichte bei 600 nm) von etwa 1,0 bei 37°C und 200 UpM inkubiert. Die Induktion der Proteinexpression erfolgte mit 1 mM IPTG bei 37°C für 6 h. Die Zellen wurden bei 10 000 × g für 10 min pelletiert und nach Entfernen des Überstandes bei -20°C bis zur Verwendung gelagert.

1.2 Zellaufschluss

Der Zellaufschluss der geernteten Zellen erfolgte in 20 mL EcUPPS-Lysepuffer (25 mM Tris, 150 mM NaCl, pH 7,5) oder ThkCPT-Lysepuffer (25 mM Tris, 150 mM NaCl, pH 8,5) versetzt mit 750 $\mu g\text{-}mL^{-1}$ Lysozym und 1 $\mu g\text{-}mL^{-1}$ DNase I zunächst enzymatisch für 1 h auf Eis auf einem Taumler bei 50 UpM. Anschließend wurde die Zellsuspension mit einem Ultraschall-Homogenisator (Amplitude: 70 %, Impuls an: 1 s, Impuls aus: 1 s) 3-mal für 30 s zusätzlich mechanisch aufgeschlossen. Das Zelllysat wurde zwischen den Homogenisationsschritten für 1 min auf Eis gekühlt.

1.3 Expression Reinigung der EcUPPS mittels Immobilisierter Metallche-lat-Affinitätschromatographie (IMAC)

Das aus 1.2 erhaltene Zelllysat wurde für 30 min bei 15 000 x g zentrifugiert, das Pellet verworfen und der Überstand mit dem löslichen, überexprimierten Protein mit Hilfe der Metallchelat-Affinitätschromatographie an Ni-Sepharose oder Co-Sepharose (TALON®) gereinigt. Die Komplexierung und damit die Bindung des Zielproteins an das IMAC-Material erfolgte über einen 6x-His-Tag.

1.4 Reinigung der EcUPPS mittel Ni-IMAC

Die Reinigung der EcUPPS erfolgte an einer FPLC-Anlage mit einer Nickel-Sepharose-Säule (HisTrap™ FF crude 1 mL, GE Healthcare, Freiburg) nach Herstellerangaben. Die cytosolische Proteinlösung wurde mit 0,3 $mL\cdot min^{-1}$ auf die Säule aufgebracht. Die Säule wurde anschließend mit 15 mL Puffer A (25 mM Tris/ HCl, 150 mM NaCl, pH 7,5) gespült, um ungebundene Proteine zu entfernen. Puffer B (25 mM Tris/ HCl, 150 mM NaCl, 300 mM Imidazol, pH 7,5) diente zur Elution des Zielproteins mit dem folgenden Gradienten in Kombination mit Puffer A und 0,3 $mL\cdot min^{-1}$ Flussrate: (SV: Säulenvolumen):

0% Puffer B → 5 SV → 0% Puffer B → 1 SV → 10% Puffer B → 2 SV → 10% → Puffer B → 1 SV → 100% Puffer B → 7 SV → 100% Puffer B

Die Proteinfraktionen wurden mittels SDS-PAGE auf Reinheit geprüft, homogene Proteinlösungen wurden vereinigt und an PD10-Säulen (Sephadex™ G-25) entsalzt. Das gereinigte Protein, wurde in 500 µL Aliquots in Assaypuffer (50 mM Hepes/ NaOH, 100 mM NaCl, 1 mM DTT, 0,5 mM $MgCl_2$, 10 % Glycerin (v/v), pH 7,5) in flüssigem Stickstoff schockgefroren und bei -20°C bis zur Verwendung gelagert. Abbildung 1 A zeigt eine Fotographie eines mit Coomassie®-Brilliant Blau gefärbten 14%igen Gels einer SDS-PAGE mit Proben aus der EcUPPS-Proteinreinigung. 1: Bakterienlysat vor der Induktion mit IPTG; 2: Bakterienlysat nach der Induktion mit IPTG; 3: unlösliche Bestandteile; 4: Bakterienlysat mit löslichen Proteinen; 5: Proteine, die nicht an die Metallaffinitätssäule gebunden haben; 6: Eluat der Affinitätschromatographie mit gereinigtem Protein; 7: entsalzte Proteinfraktion nach PD10-Säule; M: Marker.

1.5 Reinigung der ThkCPT mittels Ni-IMAC

Die Reinigung der ThkCPT erfolgte wie oben für die EcUPPS beschrieben. Es wurde die gleiche Pufferzusammensetzung verwendet, mit der Ausnahme, dass der pH-Wert der Puffer A und Puffer B auf pH 8,5 eingestellt wurde. Für den Assaypuffer wurde ein pH-Wert von 8,0 eingestellt. Abbildung 1 B zeigt eine Fotographie eines mit Coomassie®-Brilliant Blau gefärbten 10%igen Gels einer SDS-PAGE mit Proben aus der ThkCPT-Proteinreinigung. 1: Bakterienlysat vor der Induktion mit IPTG; 2: Bakterienlysat, nach der Induktion mit IPTG; 3: unlösliche Bestandteile; 4: Bakterienlysat mit löslichen Proteinen; 5: Proteine, die nicht an die Metallaffinitätssäule gebunden haben; 6: Eluat der Affinitätschromatographie mit gereinigtem Protein; 7: entsalzte Proteinfraktion nach PD10-Säule; M: Marker.

**Beispiel 2: Proteinexpression und Zellaufschluss zur Gewinnung Prenyltransferasen MtUPPS, MIUPPS und HpUPPS**

Die cDNAs der Undecaprenyldiphosphatsynthasen (UPPS) aus *Mycobacterium tuberculosis* (MtUPPS) (UniProt-Code P60479), *Micrococcus luteus* (MIUPPS) (UniProt-Code 082827) und *Helicobacterpylori* (HpUPPS) (UniProt-Code P55984) wurden bezüglich der Codon-Verwendung optimiert und synthetisiert von MWG Eurofins (MWG-Biotech AG, Ebersberg, Deutschland). Alle Konstrukte wurden kloniert in den Vektor pET28a(+) (Merck KGaA, Darmstadt, Deutschland) mit einem *N*-terminalen poly-Histidin-Tag und in chemokompetente Expressionszellen des Stammes *E.*

*coli* Bl21 (DE3) transformiert. Die Selektion der erfolgreich transformierten Zellen erfolgte über Lysogeny Broth (LB) - Agar Platten mit dem Antibiotika Kanamycin (50 μl $ml^{-1}$) und wurden anschließend im größeren Maßstab angezogen. Dafür wurde ein 350 ml Erlenmeyer-Kolben mit Terrific Broth (TB) -Medium und 50 μg $ml^{-1}$ Kanamycin verwendet. Die Zellkultur wurde bei 37°C und 300 UpM angezogen bis zu einer optischen Absorption von 0,6 bei der Wellenlänge 595 nm. Die Proteinexpression erfolgte durch Induktion mit 1 mM Isopropyl-β-d-Thiogalaktopyranosid (IPTG) für 12 Stunden bei 23°C und 210 rpm.

Für die Protein-Reinigung wurden die Zellen geerntet mittels Zentrifugation (8000 x *g*, 10 min) und anschließend resuspendiert in 20 ml Aufschluss-Puffer (50 mM Tris/HCl, 500 mM NaCl, 40 mM Imidazol, 1 mM DTT, 0,15% Triton-X 100, pH 7.5). Die homogenisiert Suspension wurde aufgeschlossen durch Hochdruckdispersion (Constant Systems Ltd., Northants, UK) bei 1000 Bar. Das Zelllysat erneut bei 16000 x *g* für 60 min zentrifugiert und der Überstand auf eine mit Nickel-Ionen beladene IMAC-Säule (HisTrap HP, 1 ml, Qiagen, Hilden, Deutschland) in Kombination mit dem ÄKTA FPLC System (GE Healthcare, Little Chalfont, UK) aufgetragen. Um das rekombinante $His_6$-getagte Fusion-Protein von der Säule zu eluieren, wurde ein linearer Gradienten verwendet, beginnend mit 100 % Aufschluss-Puffer (ohne Triton-X 100) hin zu 100 % Elutions-Puffers (50 mM Tris/HCl, 500 mM NaCl, 500 mM Imidazol, pH 7.5) über 40 min bei einem Fluss von 0,5 ml $min^{-1}$. Die Identifikation der Protein-Fraktionen erfolgt mittels Proteinbestimmung nach Bradford und die Prüfung auf Reinheit mittels Polyacrylamid Gelelektrophorese (PAGE). Die entsprechenden Proteinfraktionen wurde über eine PD-10 Säule (GE Healthcare, Little Chalfont, UK) entsalzt und anschließend bei -80°C gelagert in Assay-Puffer (50 mM Tris, 100 mM NaCl, 1 mM DTT, pH 7.5) mit zusätzlich 10 % (v/v) Glycerol.

Die Ergebnisse werden in Abbildung 2 gezeigt. Abbildung 2 zeigt Fotographien von mit Coomassie®-Brilliant Blau gefärbten 14%igen Gelen einer SDS-PAGE mit Proben aus der Proteinreinigung der UPPS aus *Micrococcus luteus* (A), *Mycobacterium tuberculosis* (B) und *Helicobacter pylori* (C); (A): MIUPPS; M: Proteingrößenstandard (10-130 kDa); 1: Rohextrakt nach Induktion mit IPTG; 2: Protein nach Metall-Affinitätschromatographie-Reinigung und Entsalzung. (B) MtUPPS: M: Proteingrößenstandard (10-130 kDa); 1: Rohextrakt nach Induktion mit IPTG; 2: Rohextrakt ohne Prenyltransferase MtUPPS nach Auftragen über die $Ni^{2+}$-Säule; 3: Protein nach Metall-Affinitätschromatographie-Reinigung und Entsalzung; (C) UPPS aus *Helicobacter pylori.* M Proteingrößenstandard (10-130 kDa); 1 Rohextrakt vor Induktion mit IPTG; 2 Rohextrakt nach Induktion und Inkubation mit IPTG; 3: Protein nach Metall-Affinitätschromatographie-Reinigung und Entsalzung.

**Beispiel 3: Bestimmung von Substratkinetiken der EcUPPS und ThkCPT**

*Cis*-Prenyltransferasen entlassen während der Reaktion Diphosphat nach jedem Kondensationsschritt. Dies ist der Ansatzpunkt, der gewählt wurde, um unabhängig von der Art des Produktes den Grundumsatz zu detektieren. Zur Bestimmung der Substratkinetik von IPP wurden 0,1 μM EcUPPS oder 1 μM ThkCPT zusammen mit 10 μM FPP und verschiedenen Konzentrationen an IPP (500 μM, 250 μM, 125 μM, 62,5 μM, 31,25 μM, 15,625 μM, 0 μM) für 72 s, 120 s, 165 s, 300 s, 521 s, 600 s und 900 s in EcUPPS-Assaypuffer (50 mM Hepes/ NaOH, 100 mM NaCl, 1 mM DTT, 0,5 mM $MgCl_2$, 10 % Glycerin (v/v), pH 7,5) oder ThkCPT-Assaypuffer (50 mM Hepes/ NaOH, 100 mM NaCl, 1 mM DTT, 1 mM $MgCl_2$, 10 % Glycerin (v/v), pH 8,0) versetzt mit 0,1 % Triton X-100 (v/v) und 8 U/mL inorganischer Phosphatase bei 22°C inkubiert. Die Dreifachbestimmungen der Enzymreaktionen wurden mit 100 μL BIOMOL Green™ Detektionsreagenz gestoppt und nach weiteren 20 min in einem Mikrotiterplattenspektrophotometer bei 620 nm gemessen. Zur Kontrolle erfolgte für jede Substratkonzentration eine Messung mit denaturiertem Enzym zu den unterschiedlichen Zeitpunkten um Selbsthydrolyse der Substrate zu berücksichtigen. Zur Quantifizierung wurde eine $P_i$-Standardkurve in Doppelbestimmung mitgeführt. Die Verdünnung des $KP_i$-Standards erfolgte in Assaypuffer, wurde mit 100 μL BIOMOL Green™ Detektionsreagenz nach Ablauf der Enzymreaktion in den Test-*Wells* detektiert und zeitgleich nach weiteren 20 min bei 620 nm gemessen. Eine lineare Beziehung zwischen $A_{620\ nm}$ und $P_i$-Konzentration ermöglichte eine Quantifizierung zwischen 80 μM-0 μM $P_i$.

Die Bestimmung der FPP-Kinetiken der EcUPPS sowie der ThkCPT erfolgte unter analogen Bedingungen der Bestimmung von IPP. Die Phosphathydrolyse wurde mit 150 μM IPP gemessen, wobei die Substratkonzentration von FPP variierte (80 μM, 40 μM, 20 μM, 10 μM, 5 μM, 2,5 μM, 1,25 μM, 0 μM). Die Enzymreaktion wurde nach 60 s, 120 s, 180 s, 300 s, 600 s, 900 s oder 1200 s durch Zugabe von 100 μL BIOMOL Green™ Detektionsreagenz gestoppt und wie oben beschrieben gemessen.

Damit konnte ein linearer Messbereich für Umsatz-Zeit-Kurven bestimmt werden, so dass Folgemessungen zu definierten Zeitpunkten gestoppt werden konnten.

**Beispiel 4: Screening der MtUPPS, MIUPPS und HpUPPS auf IPP-Umsatz und Cl-BPP-Umsatz**

Die Enzymreaktion erfolgte in 50 μl Ansätzen in Assay-Puffer mit zusätzlichen 1 mM $MgCl_2$ und 0.1 % Triton X-1 00 sowie 5 μM Farnesyldiphosphat (FPP), 50 μM Isopentenyldiphosphat (IPP) oder 50 μM Cl-BPP und 10 μM HpUPP oder MIUPPS oder MtUPPS. Die Enzymreaktion wurde durch Zugabe von 100 μl BIOMOL Green™ (ENZO

LIFE SCIENCES GmbH, Lorrach, Deutschland) in bestimmten Zeitintervallen abgestoppt und in einem Mikrotiterplattenspektrophotometer bei 620 nm gemessen. Zur Kontrolle erfolgte für jede Substratkonzentration eine Messung mit denaturiertem Enzym zu den unterschiedlichen Zeitpunkten um Selbsthydrolyse der Substrate zu berücksichtigen. Zur Quantifizierung wurde eine $P_i$-Standardkurve in Doppelbestimmung mitgeführt. Die Verdünnung des $KP_i$-Standards erfolgte in Assaypuffer, wurde mit 100 µL BIOMOL Green™ Detektionsreagenz nach Ablauf der Enzymreaktion in den Test-*Wells* detektiert und zeitgleich nach weiteren 20 min bei 620 nm gemessen. Eine lineare Beziehung zwischen $A_{620\ nm}$ und $P_i$-Konzentration ermöglichte eine Quantifizierung zwischen 80 µM-0 µM $P_i$.

**Beispiel 5: Bestimmung der Substratkinetiken artifizieller Substrate**

Die Assaybedingungen wurden wie in Beispiel 3 beschriebenen gewählt. Zur Detektion der Produktbildung wurden 0,1-1 µM *cis*-Prenyltransferase (CPT) zusammen mit verschiedenen Substraten zwischen 80 - 0 µM im Fall der Startersubstrate, variiert, wobei das Elongationssubstrat IPP mit 150 µM konstant war. Variation von 500 - 0 µM im Fall der Elongationssubstrate erfolgte konstant mit 10 µM FPP (jedes Homoallyldiphosphat möglich).

**Beispiel 6: Berechnung der Umsatzraten und kinetischen Parameter**

Zur Berechnung der Umsatzraten in $nkat{\cdot}mg^{-1}$ wurde verwendet:

Gleichung 1:

$$v_0 = \frac{(A_{620} - A_{\mathrm{Blank}}) \cdot 1000}{2 \cdot m \cdot t \cdot [E]}$$

| | |
|---|---|
| $v_0$ | Anfangsgeschwindigkeit der Diphosphathydrolyse ($nkat{\cdot}mg^{-1}$) |
| $A_{620}$ | Absorption bei 620 nm |
| $A_{Blank}$ | Absorption der Kontrolle bei 620 nm |
| m | Anstieg der Phosphatstandardkurve ($\mu M^{-1}$) |
| t | Dauer der Enzymreaktion (s) |
| [E] | Enzymkonzentration ($mg{\cdot}L^{-1}$) |

Dabei wird 1 Katal als diejenige Menge Enzym, die unter angegebenen Bedingungen 1 mol Substrat pro Sekunde umsetzt. In dem hier beschriebenen Fall handelt es sich um die indirekte Detektion einer Produktbildung über den Nachweis der Diphsophathydrolyse während der Enzymreaktion.

Die Auswertung erfolgte durch nicht-lineare Regression nach der Hill-Gleichung mit Sigma-Plot 11. Die Michaelis-Menten-Kinetik stellt einen Sonderfall der Hill-Kinetik dar, wobei h=1 ist.

Gleichung 2:

$$v = \frac{v_{\mathrm{max}} \cdot [S]^{\mathrm{h}}}{K_{\mathrm{m}} + [S]^{\mathrm{h}}}$$

| | |
|---|---|
| $K_m$ | Substratkonzentration bei halbmaximaler Geschwindigkeit der Diphosphathydrolyse (µM) |
| v | Geschwindigkeit der Diphosphathydrolyse ($nkat{\cdot}mg^{-1}$) |
| $v_{max}$ | maximale Geschwindigkeit ($nkat{\cdot}mg^{-1}$) |
| [S] | Substratkonzentration (µM) |
| h | Hill-Koeffizient |

**Beispiel 7: Bestimmung des Produktspektrums der EcUPPS und ThkCPT**

Zur Bestimmung der Produktkettenlängen wurden 500 µL Enzymassays in MS-Gläschen durchgeführt. Dazu wurden 500 µL Assaypuffer (50 mM Hepes, 100 mM NaCl, 1 mM DTT, 10 % Glycerin, 0,1 % Triton X-100, 0,5 mM $MgCl_2$ (E-cUPPS) oder 1 mM $MgCl_2$ (ThkCPT)) mit 10 µM Enzym, 50 µM Startersubstrat und 400 µM Elongationssubstrat

(Isopentenyldiphosphat (IPP), 3-Brom-3-Butenyldiphosphat (Br-BPP) bzw. 3-Chlor-3-Butenyldiphosphat (Cl-BPP)) bei Raumtemperatur (EcUPPS) oder 65 °C (ThkCPT) über Nacht inkubiert. Die Extraktion der Polyprenyldiphosphate erfolgte mit 500 μL 1-Butanol. Die Butanolphase wurde in ein neues Glas überführt und unter Stickstoffstrom von dem Lösungsmittel befreit. Die Hydrolyse der Diphosphate zum Polyprenol erfolgte durch Zugabe von 200 μL Hydrolysepuffer (20% Isopropanol, 4,4 Units/mL saure Phophatase aus Kartoffeln, 0,1 % Triton X-100, 50 mM Natriumacetat (pH 4,7)) und wurde über Nacht bei 37 °C inkubiert. Die Polyprenole wurden mit 500 μL *n*Hexan extrahiert und zur weiteren DC- oder HPLC-Analyse vorbereitet. Für DC-Experimente und LC/ESI-MS-Analysen wurde die *n*Hexan-Phase in ein neues Glas überführt, das Lösungsmittel abgedampft und die Produkte erneut in 100 μL *n*Hexan resuspendiert um eine Aufkonzentrierung zu erreichen. Für HPLC-Analysen mit **110** ((2E,6E)-8-O-(*N*-methyl-2-aminobenzoyl)-3,7-dimethyl-2,6-octandien-1-diphosphat) als Startersubstrat wurde die *n*Hexan-Phase 1:3 verdünnt in das HPLC-System mittels Autosampler injiziert.

**Beispiel 8: HPLC-Analytik der Polyprenole mit FPP als Startersubstrat**

Die HPLC-Analyse der Polyprenole erfolgte an einer Anlage von Merck HITACHI D-7000, wobei als stationäre Phase eine YMC-ODS AQ (150 x 4 mm, 5 μm; YMC Europe GmbH, Dinslaken) Säule verwendet wurde. Die Elution erfolgte mit 1 mL·min$^{-1}$ und die Detektion bei 210 nm mit einem UV-VIS Detektor: Merck HITACHI L-7420. Das Injektionsvolumen betrug dabei 5 μL. Die mobile Phase setzte sich aus Laufmittel A (MeOH/$H_2O$/Isopropanol 12:1:8 (*v*/*v*/*v*)) und Laufmittel B (nHexan/Isopropanol 7:3 (*v*/*v*)) zusammen. Die Probenaufgabe erfolgte mittels Autosampler (Merck HITACHI L-7250).

<u>Laufmittelgradient: LM - Laufmittel</u>

0 % LM B —30 min→ 45 % LM B —5 min→ 70 % LM B —1 min→ 100 % LM B

**Beispiel 9: HPLC-Analytik der Polyprenole mit 110 als Startersubstrat**

Zur selektiven und vor allem sensitiven Detektion von Polyprenolen kann ein alternatives, fluoreszierendes Startersubstrat verwendet werden, (2E,6E)-8-O-(*N*-methyl-2-aminobenzoyl)-3,7-dimethyl-2,6-octandien-1-diphosphat (110). Die Analyse erfolgte wie in Beispiel 6 beschrieben. Dabei wurden 3 μL pro Probe injiziert und die Detektion erfolgte über Fluoreszenz: Ex 352 nm / Em 420 nm wobei das Gerät Merck HITACHI L-7480 als Fluoreszenzdetektor verwendet wurde.

**Beispiel 10: LC/MS-Analyse**

Zur eindeutigen Kettenlängenbestimmung der Polyprenole wurden LC/MS-Messungen durchgeführt. Die LC-Trennung erfolgte dabei wie in Beispiel 6 beschrieben an einer HPLC-Anlage von Agilent Technologies der 1200er Serie. Dabei war das LC-System mit einem API 3200 LC/MS/MS System von AB Sciex gekoppelt. Die ESI-Spektren wurden während eines HPLC-Laufes unter einer lonensprayspannung von 4,5 kV bzw. -4,5 kV bei 400 °C unter Stickstoffstrom gemessen. Hierbei betrug das Probenvolumen 10 μL, wobei die Probe unverdünnt eingesetzt wurde.

**Beispiel 11: Bestimmung der Aktivität der Prenyltransferasen**

Die Aktivität der Prenyltransferasen wurde hinsichtlich der Umsetzung von Farnesyldiphosphat (FPP) als Startersubstrat und Isopentenyldiphosphat (IPP), und Cl-BPP bzw. Br-BPP mit Hilfe des MTP-Phosphatdetektionsassays bestimmt.

Die Reaktionen wurden in einem 50 mM Tris Puffer pH 7,5 in Gegenwart von 50 mM NaCl und 0,05% Triton X-100, 5 μM *E,E*-FPP, 200 μM IPP bzw. Cl-BPP und jeweils 50 μM Enzym bei Raumtemperatur durchgeführt. Die Ergebnisse werden in Abbildungen 3 und 4 dargestellt, wobei die Aktivität der verschiedenen *cis*-Prenyltransferasen hinsichtlich der Umsetzung von FPP mit IPP bzw. Cl-BPP gezeigt werden. Als Negativkontrolle diente ein Ansatz ohne Enzym (nicht gezeigt).

Bei Umsetzung von IPP als Elongationssubstrat wies die UPPS aus *E.coli* (EcUPPS) die höchste Aktivität auf, gefolgt von den Enzymen aus *Micrococcus luteus* (MlUPPS) und *Helicobacter pylori* (HpUPPS), wohingegen die UPPS aus *Mycobacterium tuberculosis* (MtUPPS) keine Aktivität zeigte (Abbildung 3). Dies ist auf das Verwenden von *E,E*-FPP anstatt des natürlichen Substrats *E,Z*-FPP zurück zu führen. Beim Umsatz von Cl-BPP als Elongationssubstrat zeigten die EcUPPS und die MtUPPS die höchste Aktivität. Dieses Substrat wurde im geringeren Maße auch von der UPPS

aus *Micrococcus luteus* (MlUPPS) und von der HpUPPS (HpUPPS) umgesetzt (Abbildung 4).

Diese Aktivitätstests zeigten, dass neben der EcUPPS auch andere Enzyme in der Lage waren 3-Halogen substituiertes Isopentenyldiphosphat (hier am Beispiel des Cl-BPP) als Substrate zu akzeptieren. In den nachfolgenden Beispielen werden die Versuche beschrieben, die zur Bestimmung der kinetischen Konstanten und der synthetisierten Kettenlänge durchgeführt wurden.

**Beispiel 12: Analyse des Startersubstratspektrums der EcUPPS**

Das Substratspektrum der EcUPPS wurde anhand nicht-natürlicher und natürlicher Substrate getestet. Die Aufklärung der für die Substraterkennung bedeutsamen Strukturmerkmale ist wichtig, um veränderte Substrate als Startereinheit zu verwenden. Dies ermöglicht die Einführung einer Fluoreszenzeinheit zur sensitiven und selektiven Detektion der enzymatischen Produkte oder einer Sonde für die Massenspektrometrie, da Prenyldiphosphate sowohl sehr gering absorbieren als auch ionisieren. In der Tabelle X sind alle während der für die vorliegende Anmeldung/Erfindung durchgeführten experimentellen Studien getesteten artifiziellen Substratanaloga dargestellt, die als Startereinheit für die EcUPPS dienen könnten. 18 Prenyldiphosphate konnten dabei als Substrat dienen und mit IPP als Elongationssubstrat Polyprenyldiphosphate der erwarteten Kettenlänge hervorrufen. Weitere acht Substrate wurden nicht als Startersubstrat erkannt und führten nicht zu Produktbildung mit der EcUPPS. Abbildung 5 zeigt die artifiziellen Startersubstrate der EcUPPS mit ihrer relativen katalytischen Effizienz (% $k_{cat} \cdot K_m^{-1}$) der EcUPPS mit dem jeweiligen Substrat im Bezug zu dem natürlichen Substrat FPP (100 %) abnehmend von oben nach unten. Abbildung 6 zeigt die getesteten artifiziellen Substrate, die von der EcUPPS nicht akzeptiert werden.

Mit Hilfe des MTP-Phosphatdetektionsassays wurden Substratumsatzkurven für die verschiedenen Substrate bestimmt. Dabei konnten die in Tabelle 1 dargestellten kinetischen Parameter für die getesteten Substrate gemessen werden. Der $K_m$-Wert beschreibt diejenige Substratkonzentration, bei der die halbmaximale Geschwindigkeit der Enzymreaktion erreicht ist. Dieser Wert ist ein Maß der Affinität des jeweiligen Substrates zum Enzym. Je niedriger dieser Wert ist, desto höher ist die Affinität des Substrates. Alle getesteten Substrate lagen mit ihren $K_m$-Werten im µM-Bereich. Dennoch wurden einige Substrate bis zu 30fach schlechter von dem Enzym akzeptiert. Eine schlechte Substraterkennung betraf hauptsächlich Substrate mit Heteroatomen in der terminalen Prenyleinheit **(106, 112, 113).** Das Fehlen einer terminalen Prenyleinheit, wie im Fall von GPP, hatte ebenfalls einen starken Einfluss auf die Substraterkennung. Die $K_m$-Werte von kurzkettigen Substraten wie **102** oder **107** waren niedriger als der von GPP aber hier erfolgte die Substraterkennung wahrscheinlich über den Furan- bzw. Thiophenring. Eine fünffach schlechtere Substraterkennung wiesen **105, 107** und **111** auf. Hier war wahrscheinlich auch eine verschlechterte Erkennung des Substrates durch eine hydrophilere terminale Prenyleinheit der Grund für die erhöhten $K_m$-Werte. Die maximalen Geschwindigkeiten ($v_{max}$) variieren zusätzlich stark (Tab. 1). Um die Substrate untereinander zu vergleichen, wurde daher die katalytische Effizienz $k_{cat} \cdot s^{-1}$ bestimmt, die als Maß für die Substratspezifität gilt. Vor allem Substrate, die FPP sehr ähneln wurden auch gleichsam mit ähnlicher Effizienz umgesetzt. Erstaunlich ist hierbei, dass *Z,E*-FPP trotz anderer Stereochemie genauso gut als Substrat erkannt wurde wie *E,E*-FPP obwohl anzunehmen wäre, dass die Orientierung in der aktiven Tasche eine andere ist. Starke strukturelle Veränderungen im Substrat beeinflussen auch die katalytische Effizienz. So konnte das Startersubstrat 110, trotz relativ schlechter katalytische Effizienz als Substrat genutzt werden. Es ermöglichte eine Fluorezenzdetektion (Em 352 nm/ Ex 420 nm) der enzymatischen Produkte und konnte in weiteren Versuchen erfolgreich verwendet werden.

**Tabelle 1:** Zusammenfassung der Bestimmungen kinetischer Parameter der EcUPPS mit artifiziellen Startersubstraten.

| | $K_m$ | $v_{max}$ | $k_{cat}$ | $k_{cat}\ K_m^{-1}$ | rel. $k_{cat}\ K_m^{-1}$ |
|---|---|---|---|---|---|
| **Substrat** | µM | nkat/mg | $s^{-1}$ | $s^{-1}mM^{-1}$ | % |
| **FPP** | **1,8 ± 0,1** | **17,9 ± 0,4** | **0,5** | **292** | **100** |
| Z,E-FPP | 1,5 ± 0,2 | 12,8 ± 0,6 | 0,4 | 262 | 90 |
| 100 | 2,1 ± 0,2 | 18,5 ± 0,7 | 0,5 | 260 | 89 |
| GGPP | 1,9 ± 0,1 | 14,9 ± 0,3 | 0,4 | 223 | 76 |
| 101 | 2,5 ± 0,1 | 14,9 ± 0,2 | 0,4 | 176 | 60 |
| 102 | 1,8 ± 0,2 | 6,1 ± 0,2 | 0,2 | 98 | 34 |
| 103 | 4,2 ± 0,2 | 13,7 ± 0,3 | 0,4 | 96 | 33 |
| 104 | 5,1 ± 0,6 | 11,5 ± 0,5 | 0,3 | 64 | 22 |

(fortgesetzt)

| | $K_m$ | $v_{max}$ | $k_{cat}$ | $k_{cat}\ K_m^{-1}$ | rel. $k_{cat}\ K_m^{-1}$ |
|---|---|---|---|---|---|
| **Substrat** | µM | nkat/mg | $s^{-1}$ | $s^{-1}mM^{-1}$ | % |
| 105 | 10,7 ± 1,5 | 14,4 ± 0,8 | 0,4 | 39 | 13 |
| 106 | 38,0 ± 5,0 | 33,6 ± 2,1 | 1,0 | 26 | 9 |
| 107 | 11,6 ± 2,6 | 8,9 ± 0,6 | 0,3 | 22 | 8 |
| 108 | 4,8 ± 1,0 | 2,9 ± 0,2 | 0,1 | 18 | 6 |
| 109 | 6,4 ± 1,1 | 2,9 ± 0,2 | 0,1 | 13 | 4 |
| 110 | 5,6 ± 0,8 | 1,9 ± 0,1 | 0,1 | 11 | 4 |
| 111 | 9,1 ± 1,3 | 3,5 ± 0,2 | 0,1 | 11 | 4 |
| 112 | 61,1 ± 12,8 | 18,1 ± 1,5 | 0,5 | 9 | 3 |
| 113 | 32,1 ± 4,6 | 6,3 ± 0,3 | 0,2 | 6 | 2 |
| GPP | 48,8 ± 8,3 | 11,3 ± 0,7 | 0,3 | 7 | 2 |

Sowohl aromatische Substituenten **(100, 110),** Furane **(102, 106)** oder Thiophene **(107, 111, 112)** sowie verzweigte Alkanketten **(105)** wurden als Substituenten von den Enzymen akzeptiert. Zudem zeigten Substrate mit Heteroatomen, die sich mehr als 5 C-Atome vom Diphosphat entfernt befinden, Umsatz, wenn auch mit geringerer Aktivität.

Der Vergleich der strukturellen Eigenschaften der umgesetzten Substrate zu den nicht umgesetzten Substraten, zeigte zwei Eigenschaften, die ein Prenyldiphosphat als Substrat der EcUPPS ausschließen: zum einen Heteroatome in der Nähe des Diphosphates **(115, 114, 117, 118, 119)** und zum anderen eine kurze Kettenlänge **(120,** DMAPP). Anhand von **116** ist ein weiteres Ausschlusskriterium denkbar. Entweder ist die Orientierung der Methylgruppe nahe dem Diphosphat entscheidend für die Substraterkennung, die sich bei **116** an C2 befindet und nicht wie üblich an C3 oder die nukleophile terminale Hydroxylgruppe ist dafür verantwortlich, dass keine Substraterkennung erfolgt. Anhand von **114** und **115** könnte die Hypothese aufgestellt werden, dass ein Schwefelatom in der Nähe des Diphosphates problematischer ist als die Einführung eines Sauerstoffs (vgl. **115** und **108).** Dagegen wurde in terminaler Position das sauerstoffhaltige Substrat **(113)** im Vergleich zu dem schwefelhaltigen Substrat **104** eher schlechter erkannt. Substrate mit einer fehlenden $CH_3$-Gruppe am C3 des allylischen Prenyldiphosphates konnten für die Mittelketten-Prenyltransferasen Hexa- und Heptaprenyldiphosphatsynthase als Substrat ausgeschlossen werden.

Um zu untersuchen ob das Startersubstrat die gebildeten Produktkettenlängen beeinflusst, wurden LC/ESI-MS Untersuchungen angestellt. Hierzu wurden die zu den Alkoholen hydrolysierten Produkte der EcUPPS-Assays mit IPP und den verschiedenen artifiziellen Startersubstraten zunächst über Flüssigkeitschromatographie getrennt und anschließend massenspektrometrisch gemessen. Dabei fiel auf, dass die Prenylalkohole bevorzugt als Natrium- und Kaliumadukte auftreten. In Tabelle 2 sind die gemessenen Masse-zu-Ladungsverhältnisse dargestellt mit den dazugehörigen IPP-Elongationsschritten. Wie zu erwarten war, können Startersubstrate, die kurzkettiger sind als FPP mehr IPP-Kondensationen hervorrufen (z.B. GPP, **102, 107)** wo hingegen längere Startersubstrate auch weniger IPP-Kondensationen bewirken (z.B. GGPP). Dies könnte auf die Tatsache zurück zu führen sein, dass die Kettenlänge der Produkte durch das Enzym terminiert wurde und somit bei kurzen Startereinheiten erst nach mehr Elongationsschritten die Synthese abbrach, während die Kettenverlängerung bei langen Startersubstraten früher stoppte. Jedoch war besonders bei längeren Startereinheiten wie z. B. GGPP auch eine Verlängerung um 8 IPP-Einheiten zum $C_{60}$-Polyprenyldiphosphat möglich. Daher war bei fast allen Startersubstraten das $C_{55}$-Produktanalogon zu finden, welches von der Größe Undecaprenyldiphosphat (UPP) entspricht. Somit können zur Produktsynthese zum Teil sieben, acht oder neun Kondensationen von IPP an das Startersubstrat erforderlich sein. Letztlich entsteht eine Verteilung ähnlicher Oligomere mit einem ausgeprägten Peak des $C_{55}$-Produktanalogons. **102** und **106** bildeten kein $C_{55}$-Produktanalogon sondern eher das $C_{65}$-Produktanalogon. Die Ursache dafür bleibt relativ unklar. Die einzige Gemeinsamkeit beider Substrate ist der Furanring, der das Substrat terminiert und möglicherweise sehr platzsparend ist und damit weitere Verlängerungen bis $C_{65}$ zuläßt. Mit **110** werden dieselben Kettenlängen erzielt wie mit dem natürlichen Substrat *E,E*-FPP. Dieses Ergebnis war wichtig, um dieses Substrat in der Analytik einzusetzen. Es gewährleistete damit Produktkettenlängen zu erreichen, die mit den erzielten Kettenlängen mit *E,E*-FPP vergleichbar sind.

**Tabelle 2.** Produktanalyse der EcUPPS Assays mit artifiziellen und natürlichen Startersubstraten sowie IPP als Elongationssubstrat mittels LC/ESI-MS. *m/z* =- Masse-zu-Ladungsverhältnis

| *Substrat* | $C_{45}$-Analogon *m/z* | IPP-Elongation # | $C_{50}$-Analogon *m/z* | IPP-Elongation # | $C_{55}$-Analogon *m/z* | IPP-Elongation # | $C_{60}$-Analogon *m/z* | IPP-Elongation # | $C_{65}$-Analogon *m/z* | IPP-Elongation # |
|---|---|---|---|---|---|---|---|---|---|---|
| E,E-FPP | 653,6 ($M+Na^+$) | **6** | 721,7 ($M+Na^+$) | **7** | 789,6 ($M+Na^+$) | **8** | 857,8 ($M+Na^+$) | **9** | - | |
| *Z,E*-FPP | - | | - | | 789,7 ($M+Na^+$)<br>805.2 ($M+K^+$) | **8** | 857,8 ($M+Na^+$) | **9** | | |
| 100 | - | | - | | 829,5 ($M+Na^+$)<br>845,6 ($M+K^+$) | **8** | 897,5 ($M+Na^+$)<br>913,6 ($M+K^+$) | **9** | - | |
| GGPP | - | | - | | 789,6 ($M+Na^+$) | **7** | 857,7 ($M+Na^+$) | **8** | - | |
| 101 | - | | 693,9 ($M+Na^+$) | **7** | 761,8 ($M+Na^+$) | **8** | 829,9 ($M+Na^+$) | **9** | - | |
| 102 | - | | - | | - | | 869,5 ($M+Na^+$)<br>885,7 ($M+K^+$) | **10** | - | |
| 103 | - | | - | | 773,6 ($M+Na^+$) | **8** | - | | - | |
| 104 | - | | 821,7 ($M+Na^+$) | **7** | 889,8 ($M+Na^+$) | **8** | - | | - | |
| 105 | - | | - | | 851,8 ($M+Na^+$) | **8** | 920,1 ($M+Na^+$) | **9** | - | |
| 106 | | | - | | - | | 883,9 ($M+Na^+$)<br>899,6 ($M+K^+$) | **9** | 951,7 ($M+Na^+$)<br>967,9 ($M+K^+$) | **10** |
| 107 | - | | - | | 817, 7 ($M+Na^+$) | **9** | - | | - | |
| 108 | - | | - | | 821,6 ($M+K^+$) | **8** | 873,6 ($M+Na^+$)<br>889,6 ($M+K^+$) | **9** | - | |
| 109 | - | | - | | 759,5 ($M+Na^+$)<br>775,5 ($M+K^+$) | **8** | 827,7 ($M+Na^+$)<br>843,4 ($M+K^+$) | **9** | - | |
| 110 | - | | 802,5 ($M+Na^+$) | **7** | 870,6 ($M+Na^+$)<br>886,6 ($M+K^+$) | **8** | 938,7 ($M+Na^+$)<br>954,5 ($M+K^+$) | **9** | - | |
| 111 | - | | - | | 845,5 ($M+Na^+$)<br>861,5 ($M+K^+$) | | 913,5 ($M+Na^+$)<br>929,6 ($M+K^+$) | | - | |
| 112 | - | | - | | 831,9 ($M+Na^+$)<br>847,8 ($M+K^+$) | **8** | 899,5 ($M+Na^+$)<br>915,5 ($M+K^+$) | **9** | 967,6 ($M+Na^+$)<br>983,7 ($M+K^+$) | **10** |

(fortgesetzt)

| *Substrat* | $C_{45}$-Analogon *m/z* | IPP-Elongation # | $C_{50}$-Analogon *m/z* | IPP-Elongation # | $C_{55}$-Analogon *m/z* | IPP-Elongation # | $C_{60}$-Analogon *m/z* | IPP-Elongation # | $C_{65}$-Analogon *m/z* | IPP-Elongation # |
|---|---|---|---|---|---|---|---|---|---|---|
| 113 | - | | - | | 873,8 ($M+Na^+$) | **8** | 941,9 ($M+Na^+$) | **9** | - | |
| GPP | - | | - | | 789,4 ($M+Na^+$) 805,6 ($M+K^+$) | **9** | - | | - | |

**Beispiel 13: Analyse artifizieller Elongationssubstrate der EcUPPS**

Mit den beiden Mittelketten-cis-Prenyltransferasen Undecaprenyldiphosphatsynthase aus *E. coli* (EcUPPS) und der *cis*-Prenyltransferase aus *Thermococcus kodakaraensis* (ThkCPT) wurden verschiedene IPP-Analoga zunächst im Mikrotiterplattenformat getestet. Substrate, die von den Enzymen konvertiert werden konnten, wurden anschließend über DC, HPLC und LC/ESI-MS näher charakterisiert. Als Elongationssubstrate wurden IPP als natürliches Substrat sowie die artifiziellen Substrate 3-Chlor-Butenyldiphosphat (Cl-BPP) und 3-Brom-Butenyldiphosphat (Br-BPP) eingesetzt.

Die Analyse der artifiziellen Elongationssubstrate (Cl-BPP und Br-BPP) mit der EcUPPS erfolgte mit Hilfe des MTP-Phosphatdetektionsassays (Beispiel 3). Tabelle 3 zeigt die katalytische Effizienz der Enzyme mit den Substraten.

**Tabelle 3:** Bestimmung katalytischer Parameter der EcUPPS mit FPP und den artifiziellen Elongationssubstraten Chlor-Butenyldiphosphat (Cl-BPP) und Brom-Butenyldiphosphat (Br-BPP), im Vergleich zu Isopentenyldiphophat (IPP).

| | IPP | Cl-BPP | Br-BPP |
|---|---|---|---|
| $K_m$ ($\mu$M) | 6,5 +/- 0,4 | 3,8 +/- 0,5 | 6,4 +/- 0,6 |
| $v_{max}$ (nkat $mg^{-1}$) | 14,4 | 0,03 | 0,02 |
| $k_{cat}$ ($s^{-1}$) | 0,4 | 0,0009 | 0,0004 |

Von besonderer Bedeutung war die mit den Elongationssubstraten gebildete Kettenlänge. Hierzu erfolgten Reaktionen in MS-Gläschen mit 10 $\mu$M EcUPPS, **110** als Startersubstrat sowie dem jeweiligen Elongationssubstrat. Wie bereits bei der Analyse der Startereinheiten geklärt werden konnte, ist die gebildete Kettenlänge nicht von dem Startersubstrat abhängig. Im Anschluss an die Enzymreaktion mit der EcUPPS wurden die Diphosphate der Produkte mit Hilfe einer sauren Phosphatase aus Kartoffeln zum Prenylalkohol hydrolysiert, um eine Analyse über HPLC zu ermöglichen. Alle anschließend gezeigten enzymatischen Produkte sind Phosphathydrolyseprodukte des eigentlich gebildeten Polyprenyldiphosphats.

Mittels HPL-Chromatogramme der Produkte, die während der Enzymreaktion der EcUPPS mit **110** als Startersubstrat und IPP, Br-BPP und Cl-BPP entstanden konnte mit IPP eine Kondensation von 8-9 Elongationseinheiten beobachtet werden. Für die artifiziellen Elongationssubstrate Cl-BPP und Br-BPP konnten 2 Kondensationsschritte nachgewiesen werden.

Die weitere Analyse der entstanden Produktkettenlängen erfolgte über Dünnschichtchromatographie (DC), wobei die Ergebnisse der HPLC-Analyse bestätigt werden konnten. Für die Dünnschichtchromatographie wurden EcUPPS-Assays mit FPP als Startersubstrat durchgeführt. Bei der DC-Analytik wurde beobachtet, dass die **110**-Produkte durch den Fluorophor ein etwas anderes Laufverhalten aufwiesen. Die Trennung der Produkte mit FPP als Startereinheit war dagegen sehr gut und unproblematisch.

Für die Cl- und Br-BPP-Produkte konnte ein Nachweis der Produkte über DC erfolgen. Hier sind jeweils zwei Produktspots im Vergleich zu der Kontrolle mit denaturiertem Enzym zu erkennen (Daten nicht gezeigt). Es handelt sich dabei um das einfach- und zweifachkondensierte Reaktionsprodukt mit dem Startersubstrat FPP.

Der Nachweis zweifachkondensierter Cl- und Br-BPP-Produkte mit 110 konnte über LC/ESI-MS geführt werden. Dabei wurde mit einer Retentionszeit von 2,25 min und einem *m/z* = 502,1 $[M+Na^+]$ (berechnet: *m/z* = 502,2 $[M+Na^+]$) das Dichlorprodukt mit **110** und mit einer Retentionszeit von 2,44 min und *m/z* = 591,7 $[M+Na^+]$ (berechnet: *m/z*=592,1 $[M+Na^+]$) das zweifachbromierte **110**-Produkt nachgewiesen. Für die Produktbildung von **110** mit IPP als Elongationssubstrat konnte eine Produktverteilung von sieben bis neun Elongationen nachgewiesen werden. Zusätzlich konnte beobachtet werden, dass alle Produkte auch als Kaliumaddukt $[M+K^+]$ auftreten, was als zusätzlicher Nachweis der Molekülionen dienen kann.

Die Ergebnisse der Analysen werden in Tabelle 4 zusammengefasst.

**Tabelle 4:** LC/ESI-MS Analyse der **110**-Produkte der EcUPPS mit IPP, Cl-BPP und Br-BPP. m/z =, Masse-zu-Ladungsverhältnis; RT, Retentionszeit (min); VL, Verlängerung(en).

| | 1 VL | 2 VL | 7 VL | 8 VL | 9 VL |
|---|---|---|---|---|---|
| Elongationssubstrat | *m/z* $[M+Na]^+$ | *m/z* $[M+Na]^+$ | *m/z* $[M+Na]^+$ | *m/z* $[M+Na]^+$ | *m/z* $[M+Na]^+$ |
| IPP | - | - | 802,4 (RT 8,77) | 870,5 (RT 10,94) | 938,7 (RT 13,27) |

(fortgesetzt)

| | 1 VL | 2 VL | 7 VL | 8 VL | 9 VL |
|---|---|---|---|---|---|
| Elongationssubstrat | *m*/*z* $[M+Na]^+$ | *m*/*z* $[M+Na]^+$ | *m*/*z* $[M+Na]^+$ | *m*/*z* $[M+Na]^+$ | *m*/*z* $[M+Na]^+$ |
| Cl-BPP | - | 502,1 (RT 2,25) | - | - | - |
| Br-BPP | - | 591,7 (RT 2,44) | - | - | - |

Somit ist es gelungen mit vier verschiedenen Analysemethoden (DC, HPLC und LC/ESI-MS) die Produktkettenlängen der artifiziellen Polyprenole als auch der natürlichen EcUPPS-Produkte zu bestimmen. Die Kombination aus DC, HPLC und LC/ESI-MS stellt sich als sehr zuverlässig heraus. Der Nachweis über LC erfolgte mit anschließender Elektrosprayionisierung im Positivionenmodus . Die Polyprenole treten bevorzugt als Natriumaddukte des Moleküls auf, doch auch Protonen- und Kaliumaddukte sind oft sichtbar. Die HPLC-Methode mit dem fluoreszierenden **110** als Startersubstrat ist eine sehr sensitive Methode, die selbst sehr geringe Produktmengen detektieren kann. Durch die Fluoreszenzdetektion ist sie eine gleichsam sehr selektive Methode, die es ermöglicht ausschließlich enzymatische Produkte zu analysieren. Die Dünnschichtchromatographie ermöglicht zusätzlich eine sehr schnelle und einfache Vorbestimmung der Produkte. Somit steht hier ein sehr schnelles, redundantes, selektives und sensitives Analysespektrum zur Verfügung um die Kettenlängen der Produktalkohole zu analysieren.

**Beispiel 14: Analyse des Elongationssubstratspektrums der ThkCPT**

Die Analyse des Elongationssubstratspektrums der ThkCPT erfolgte in derselben Weise wie in Beispiel 13 für die EcUPPS beschrieben. Die Analyse der artifiziellen Elongationssubstrate (Cl-BPP und Br-BPP) mit der ThkCPT erfolgte mit Hilfe des MTP-Phosphatdetektionsassays (Beispiel 3). Tabelle 5 zeigt die katalytische Effizienz der Enzyme mit den Substraten. Die nicht-natürlichen Elongationssubstrate wurden von diesem Enzym schlechter umgesetzt als IPP. Die Umsatzraten lagen in demselben Bereich wie mit der EcUPPS und waren in diesem Fall 150-200fach geringer als mit IPP. Die $K_m$-Werte lagen im Bereich des Wertes von IPP.

**Tabelle 5:** Bestimmung katalytischer Parameter der ThkCPT mit FPP und den artifiziellen Elongationssubstraten Chlor-Butenyldiphosphat (Cl-BPP) und Brom-Butenyldiphosphat (Br-BPP), im Vergleich zu Isopentenyldiphophat (IPP).

| | IPP | Cl-BPP | Br-BPP |
|---|---|---|---|
| $K_m$ ($\mu$M) | 14,8 +/- 1,4 | 21,2+/-2,1 | 54,0 +/- 11,1 |
| $v_{max}$ (nKat $mg^{-1}$) | 2,0 | 0,01 | 0,01 |
| $k_{cat}$ ($s^{-1}$) | 0,07 | 0,0005 | 0,0004 |

Da der MTP-Phosphatassay bei Raumtemperatur durchgeführt wurde, war sehr wichtig die Produktbildung unter optimalen Reaktionsbedingungen zu kontrollieren. Dazu wurden HPLC-Analysen durchgeführt. Die Enzymassays wurden dazu bei 65°C mit dem Fluoreszenzsubstrat **110** sowie dem jeweiligen Elongationssubstrat inkubiert und anschließend über HPLC analysiert (Abbildung 8). Abbildung 8 zeigt ein HPL-Chromatogramm der Produkte der ThkCPT mit **110** und den Elongationssubstraten Br-BPP und Cl-BPP. Die Detektion erfolgte über Fluoreszenz bei Ex 352 nm/Em 420 nm. Eingesetzt wurde 10 $\mu$M ThkCPT. **110** wurde von dem thermophilen Enzym ThkCPT ebenfalls als Startersubstrat akzeptiert und die Produktbildung konnte analysiert werden. Mit IPP als Elongationssubstrat wurde eine Produktverteilung mittelkettiger **110-**Produktanaloga von $C_{45}$-$C_{65}$ erhalten. Mit der ThkCPT wurde aus **110** und dem jeweiligen artifiziellen Elongationssubstrat (Cl-BPP, Br-BPP) zum größeren Teil das einfachkondensierte Reaktionsprodukt gebildet. Dieses Reaktionsprodukt eluierte kurz hinter dem Prenylalkohol von **110,** der auch im Blank zu beobachten war. Mit den Elongationssubstraten wurden jedoch auch mit Cl-BPP und Br-BPP **110**-Produktanaloga mit höherer Kettenlänge gebildet, und zwar mindestens nachweisbar bis zum $C_{40}$ Produktanalogon.

Weiterhin wurde die Frage untersucht, ob die längeren Produktketten im Vergleich zur EcUPPS auf einen kinetischen Effekt zurückzuführen sein könnten. Um dies zu testen, wurden die HPLC-Assays in gleicher Weise wiederholt und bei Raumtemperatur inkubiert. Nach erfolgter Aufarbeitung wurden die Proben über HPLC analysiert (Abbildung 7). Abbildung 7 zeigt ein HPL-Chromatogramm der Produkte der ThkCPT mit **110** und den Elongationssubstraten Br-BPP und Cl-BPP. Die Detektion erfolgte über Fluoreszenz bei Ex 352 nm/Em 420 nm. Eingesetzt wurde 10 $\mu$M ThkCPT.

Wie schon bei der 65°C-Inkubation der ThkCPT-Assays zu beobachten war, konnten auch bei Raumtemperatur

Reaktionsprodukte mit mehr als zwei Kondensationen mit Cl-BPP bzw. Br-BPP beobachtet werden. Da die Reaktionstemperatur für die ThkCPT mit 25°C nicht optimal war, wurden die Produkte mit geringerer Abundanz gemessen. Die Ergebnisse ließen darauf schließen, dass die abgebrochene Kettenverlängerung nicht auf einen klassischen kinetischen Effekt allein zurückzuführen war. Weiterhin zeigten die Ergebnisse überraschenderweise, dass bei 25°C Produkte mit längeren Ketten entstanden, was auch bei dem natürlichen Substrat IPP zu beobachten war. Dominierend war bei den artifiziellen Substraten jedoch, gleichsam wie bei der Inkubation bei 65°C, das einfachkondensierte Reaktionsprodukt.

Anschließend wurden zur DC-Analyse Assays mit FPP und dem jeweiligen artifiziellen Elongationssubstrat bei 65°C durchgeführt, um die Produktkettenlängen zu verifizieren. Auch hier konnten das einfachkondensierte Reaktionsprodukt sowie die mehrfachkondensierten Reaktionsprodukte nachgewiesen werden (Daten nicht gezeigt).

**Beispiel 15: Erzeugung von Co-Polymeren**

Zur Erzeugung von Co-Polymeren wurden 500 μL Enzymassays in MS-Gläschen durchgeführt. Dazu wurden 500 μL Assaypuffer (50 mM Hepes, 100 mM NaCl, 1 mM DTT, 10 % Glycerin, 0,1 % Triton X-100, 0,5 mM $MgCl_2$ (EcUPPS) oder 1 mM $MgCl_2$ (ThkUPPS)) mit 10 μM Enzym, 50 μM **110** und 400 μM Elongationssubstratmix (1:0, 1:10, 1:100, 1:500, 1:1000 (IPP:Elongationssubstrat)) bei 22°C (EcUPPS) oder 65°C (ThkCPT) über Nacht inkubiert. Die Extraktion der Polyprenyldiphosphate erfolgte mit 500 μL 1-Butanol. Die Butanolphase wurde in ein neues Glas überführt und unter Stickstoffstrom von dem Lösungsmittel befreit. Die Hydrolyse der Diphosphate zum Polyprenol erfolgte durch Zugabe von 200 μL Hydrolysepuffer (20% Isopropanol, 4,4 Units/mL azide Phophatase aus Kartoffel, 0,1 % Triton X-1 00, 50 mM Natriumacetat (pH 4,7)) und wurde über Nacht bei 37 °C inkubiert. Die Polyprenole wurden mit 500 μL *n*Hexan extrahiert. Die *n*Hexan-Phase wurde 1:3 (*v*/*v*) mit *n*Hexan verdünnt in die HPLC mittels Autosampler injiziert.

Die Ergebnisse werden in Abbildung 9 dargestellt. Abbildung 9 zeigt HPL-Chromatogramme der Produkte der ThkCPT mit **110** und den Elongationssubstraten IPP, sowie Cl-BPP und Br-BPP jeweils im Gemisch mit IPP im Verhältnis 1:10 (IPP:Cl-BPP bzw. Br-BPP). Die Detektion erfolgte über Fluoreszenz bei Ex 352 nm/Em 420 nm.

Im oberen Chromatogramm der Abbildung 9 sind die Ergebnisse der Ansätze zu sehen, bei denen IPP als Elongationssubstrat verwendet wurde. Die Hauptprodukte eluieren bei etwa 17, 19 und 21 min. Die weiteren Ergebnisse zeigen, dass bei steigender Konzentration von IPP im Verhältnis zu den künstlichen Substraten, die Kettenlänge im Vergleich zu den Ansätzen mit Cl-BPP (mittleres Chromatogramm) bzw. mit Br-BPP (unteres Chromatogramm) vergrößert wurde: das Hauptprodukt, das bei Einsatz der Substratgemische (IPP/Cl-BPP und IPP/ Br-BPP) entstand, eluierte bei 13 min, während die Produkte, die bei Verwendung von reinem Cl-BPP bzw. Br-BPP entstanden (Abbildungen 7 und 8), bei etwa 3 bis 9 min eluierten. Weiterhin zeigt auch das Entstehen von Doppel- bzw. Mehrfachpeaks, dass in die Polymerkette ein Einbau von künstlichem Substrat und von IPP erfolgte, und somit im Elongationsbereich ein Co-Polymer entstanden ist. Diese mehrfachen Peaks sind bei dem Ansatz mit Cl-BPP/IPP insbesondere beim Hauptprodukt (Doppelpeak bei 13 min) aber auch bei den Produkten kürzerer Kettenlänge (6-12 min) an der Vielzahl der Peaks zu erkennen. Bei dem Ansatz mit Br-BPP/IPP sind diese Doppel- bzw. Mehrfachpeaks insbesondere bei den Produkten sichtbar, die bei 6, 7 und 9 min. eluieren.

**Patentansprüche**

**1.** Ein Verfahren zur enzymatischen Verkettung von Monomeren enthaltend die Schritte:

a) Verketten von Monomeren mit der Formel (I)

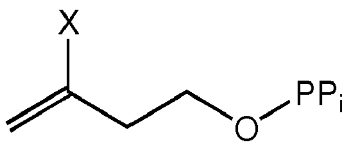

(I),

wobei X ein Halogenatom und O-$PP_i$ eine Diphosphatgruppe ist,
und optional von Isopentenyldiphosphat als weiterem Monomer,
wobei die Monomere mit einer Prenyltransferase und einem allylischen Diphosphat als Startersubstrat in Anwesenheit zweiwertiger Ionen in Kontakt gebracht werden;
b) Erhalten von Produkten mit Molekülketten, die Monomereinheiten enthalten, die durch die Formel (II) dargestellt werden:

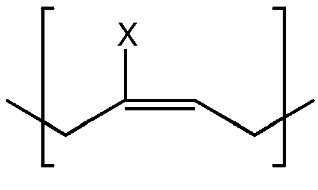

(II)

wobei X wie oben definiert ist, und
optional, wobei die Molekülketten zusätzlich Monomereinheiten enthalten, die durch die Formel (III) dargestellt werden:

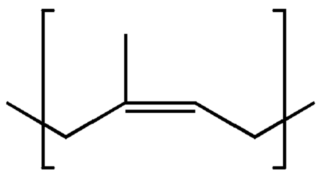

(III)

c) optional, Abspalten der Diphosphatgruppen von den in b) erhaltenen Produkten;
mit der Maßgabe, dass, wenn Isopentenyldiphosphat nicht als weiteres Monomer in der Reaktion eingesetzt wird

- und wenn X Brom ist, die Produkte mit Molekülketten mehr als 2 Monomereinheiten mit der Formel (II) aufweisen;
- und wenn X Chlor ist, die Produkte mit Molekülketten mehr als 1 Monomereinheit mit der Formel (II) aufweisen;

und
mit der Maßgabe, dass, wenn Isopentenyldiphosphat als weiteres Monomer in der Reaktion eingesetzt wird, der Anteil der Produkte mit Molekülketten, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht beträgt.

**2.** Ein Verfahren zur enzymatischen Herstellung von Produkten durch Umsetzen eines Reaktionsgemisches enthaltend allylisches Diphosphat als Startersubstrat und 3-Halo-3-Butenyl-1-Diphosphat als Monomer zur Elongation in Anwesenheit einer Prenyltransferase und zweiwertiger Ionen, wobei optional zusätzlich Isopentenyldiphosphat als weiteres Monomer zur Elongation in dem Reaktionsgemisch enthalten sein kann mit den Maßgaben nach Anspruch 1.

**3.** Ein Verfahren zur enzymatischen Herstellung von Produkten, insbesondere gemäß Anspruch 2, enthaltend die Schritte:

a) Umsetzen eines Reaktionsgemisches enthaltend allylisches Diphosphat als Startermolekül und Monomere mit der Formel (I) als Elongationssubstrat

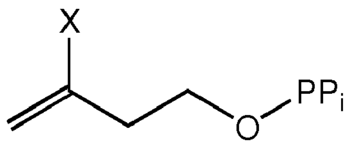

(I),

wobei X ein Halogenatom und O-$PP_i$ eine Diphosphatgruppe ist,
und optional von Isopentenyldiphosphat als weiterem Monomer, in Anwesenheit einer Prenyltransferase und zweiwertiger Ionen;
b) Erhalten von Produkten mit Molekülketten, die Monomereinheiten enthalten, die durch die Formel (II) dargestellt werden:

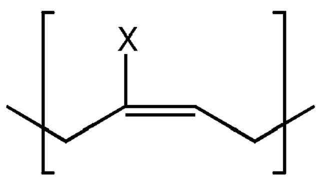

(II)

wobei X wie oben definiert ist, und
optional, wobei die Molekülketten zusätzlich Monomereinheiten enthalten, die durch die Formel (III) dargestellt werden:

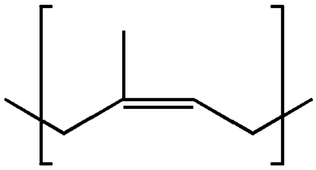
(III)

c) optional: Abspalten der Diphosphatgruppe von den in b) erhaltenen Produkten;
mit den Maßgaben gemäß Anspruch 1.

**4.** Das Verfahren gemäß irgendeinem der Ansprüche 1 bis 3, wobei Produkte mit der Formel (IV) erhalten werden

$R^1$-$A_n$-$B_m$-O-$PP_i$ (IV),

wobei $R^1$ der Rest des allylischen Startersubstrats ist, A die Monomereinheit [$CH_2$-C(X)=CH-$CH_2$] und B die Monomereinheit [$CH_2$-C($CH_3$)=CH-$CH_2$] darstellt, wobei X ein Halogenatom, O-$PP_i$ eine Diphosphatgruppe oder durch O-H ersetzt ist, n eine ganze Zahl von 1 bis 30000 und m eine ganze Zahl von 0 bis 30000 ist, wobei die Summe von n und m eine ganze Zahl von 1 bis 30000 ist,
wobei, wenn m nicht 0 ist, die Monomereinheiten A und B in ihrer Anordnung statistisch verteilt sind oder A-O-$PP_i$ endständig angeordnet ist,
mit der Maßgabe, dass, wenn m = 0 ist, und

- wenn X ein Bromatom ist, n größer als 2 ist oder
- wenn X ein Chloratom ist, n größer als 1 ist.

**5.** Das Verfahren gemäß irgendeinem der Ansprüche 1 bis 4, wobei zusätzlich Isopentenyldiphosphat als weiteres Monomer bei der Reaktion anwesend ist.

**6.** Das Verfahren gemäß irgendeinem der Ansprüche 1 bis 5, wobei Produkte mit Molekülketten erhalten werden, die in denselben Molekülketten Monomereinheiten der Formel (II) und (III) enthalten:

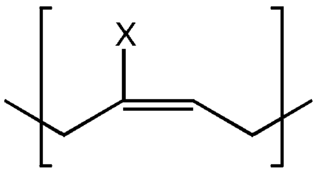
(II),

wobei X ein Halogenatom ist,
und

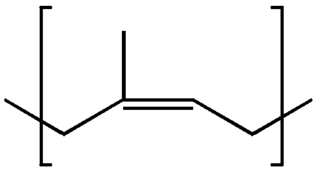
(III).

**7.** Das Verfahren gemäß irgendeinem der Ansprüche 1 bis 5, wobei Isopentenyldiphosphat bei der Reaktion nicht verwendet wird und die Produkte Monomereinheiten enthalten, die durch die Formel (II) dargestellt werden:

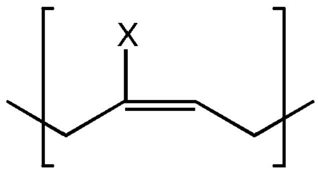

(II),

wobei X ein Halogenatom ist.

8. Das Verfahren gemäß irgendeinem der Ansprüche 1 bis 7, wobei mindestens bei 0,1 Gew.-%, bevorzugt mindestens 1 Gew.-% bezogen auf das Trockengewicht der Produkte die Zahl der Monomereinheiten eine ganze Zahl von 2 bis 30000, 2 bis 10000, 2 bis 5000, 2 bis 1000, 2 bis 500, 2 bis 100, 2 bis 50, 2 bis 30, 2 bis 20, 2 bis 10, 2 bis 8, 3 bis 30000, 3 bis 10000, 3 bis 5000, 3 bis 1000, 3 bis 500, 3 bis 100, 3 bis 50, 3 bis 30, 3 bis 20, 3 bis 10, 3 bis 8 ist.

9. Das Verfahren gemäß irgendeinem der Ansprüche 1 bis 8, wobei X ausgewählt ist aus der Gruppe bestehend aus Fluor, Chlor, Brom und Iod.

10. Das Verfahren gemäß irgendeinem der Ansprüche 1 bis 9, wobei die Prenyltransferase eine *cis*-Prenyltransferase ist.

11. Das Verfahren gemäß irgendeinem der Ansprüche 1 bis 10, wobei die Prenyltransferase eine Prenyldiphosphatsynthase ist, die **dadurch gekennzeichnet ist, dass** diese bei Verwendung des Elongationssubstrates Isoprenyldiphosphat und Farnesyldiphosphat als Startersubstrat Produkte mit einer Kettenlänge von mindestens $C_{20}$ synthetisieren, vorzugsweise mit einer Kettenlänge von $C_{20}$ bis $C_{150000}$, bevorzugt mit einer Kettenlänge von $C_{20}$ bis $C_{5000}$, weiter bevorzugt mit einer Kettenlänge von $C_{20}$ bis $C_{120}$, weiter bevorzugt mit einer Kettenlänge von $C_{20}$ bis $C_{90}$, besonders bevorzugt mit einer Kettenlänge von $C_{20}$ bis $C_{85}$. insbesondere bevorzugt mit einer Kettenlänge von $C_{20}$ bis $C_{65}$, und am meisten bevorzugt mit einer Kettenlänge von $C_{20}$ bis $C_{55}$.

12. Das Verfahren gemäß irgendeinem der Ansprüche 1 bis 11, wobei die Prenyltransferase ausgewählt aus der Gruppe bestehend aus *E. coli* Undecaprenyldiphosphatsynthase (EcUPPS, EC: 2.5.1.31; SEQ ID NO: 1), *Thermococcus kodakaraensis cis*-Prenyltransferase (ThkCPT, EC: 2.5.1.87; SEQ ID NO: 2), *Micrococcus luteus* Undecaprenyldiphosphatsynthase (MlUPPS, EC: 2.5.1.31; SEQ ID NO: 3), *Mycobacterium tuberculosis* Undecaprenyldiphosphatsynthase (MtUPPS, EC: 2.5.1.86 und 2.5.1.87; SEQ ID NO: 4), *Helicobacter pylori* Undecaprenyldiphosphatsynthase (HpUPPS, EC: 2.5.1.31; SEQ ID NO: 5), menschliche Dehydrodolichyldiphosphat-Synthase (EC: 2.5.1.87; SEQ ID NO: 6), und Kautschuk-Synthasen, insbesondere bevorzugt aus *Hevea brasiliensis* (EC 2.5.1.20; SEQ ID NO: 7) und *Taraxacum koksaghyz* (EC 2.5.1.20; SEQ ID NO: 8), Dehydrodolichyldiphosphat-Synthase aus *Arabidopsis thaliana* (EC 2.5.1.87; SEQ ID NO: 9) eingesetzt.

13. Das Verfahren gemäß irgendeinem der Ansprüche 1 bis 12, wobei das allylische Diphosphat 1,1-Dimethylallyldiphosphat, Geranyldiphosphat, Farnesyldiphosphat, Geranylgeranyldiphosphat, Neryldiphosphat oder eine Verbindung ist, die eine allylische Diphosphatendgruppe oder ein funktionalisiertes allylisches Diphosphat enthält, insbesondere (2E,6E)-8-O-(*N*-methyl-2-aminobenzoyl)-3,7-dimethyl-2,6-octandien-1-diphosphat (110).

14. Produkte mit der Formel (IV):

$$R^1\text{-}A_n\text{-}B_m\text{-}O\text{-}PP_i \qquad (IV),$$

wobei $R^1$ der Rest eines allylischen Startersubstrats ist, A die Monomereinheit $[CH_2\text{-}C(X)\text{=}CH\text{-}CH_2]$ und B die Monomereinheit $[CH_2\text{-}C(CH_3)\text{=}CH\text{-}CH_2]$ darstellt, wobei X ein Halogenatom, O-$PP_i$ eine Diphosphatgruppe oder durch O-H ersetzt ist, n eine ganze Zahl von 1 bis 30000 und m eine ganze Zahl von 0 bis 30000 ist, wobei die Summe von n und m eine ganze Zahl von 1 bis 30000 ist,
wobei, wenn m nicht 0 ist, die Monomereinheiten A und B in ihrer Anordnung statistisch verteilt sind oder A-O-$PP_i$ endständig angeordnet ist,
mit der Maßgabe, dass, wenn m = 0 ist, und

- wenn X ein Bromatom ist, n größer als 2 ist oder
- wenn X ein Chloratom ist, n größer als 1 ist.

**15.** Produkte gemäß Anspruch 14, wobei m = 0 ist.

**16.** Produkte mit der Formel (V)

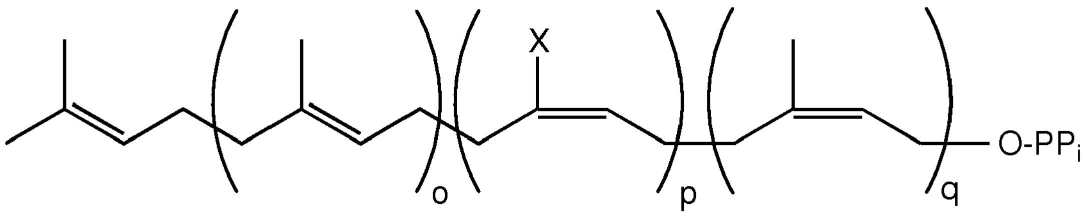

(V),

wobei X ein Halogenatom, O-$PP_i$ eine Diphosphatgruppe oder durch O-H ersetzt ist und o eine ganze Zahl von 1 bis 6, p eine ganze Zahl von 1 bis 30000 und q eine ganze Zahl von 0 bis 30000 ist, wobei die Summe von p und q eine ganze Zahl von 1 bis 30000 ist, und
wobei, wenn q nicht 0 ist, die mit p und q bezeichneten Monomereinheiten in ihrer Anordnung statistisch verteilt sind oder die mit p bezeichnete Monomereinheit zusammen mit O-$PP_i$ endständig angeordnet ist,
mit der Maßgabe, dass, wenn q = 0 ist, und

- wenn X ein Bromatom ist, p größer als 2 ist oder
- wenn X ein Chloratom ist, p größer als 1 ist.

**17.** Produkte mit der Formel (VI)

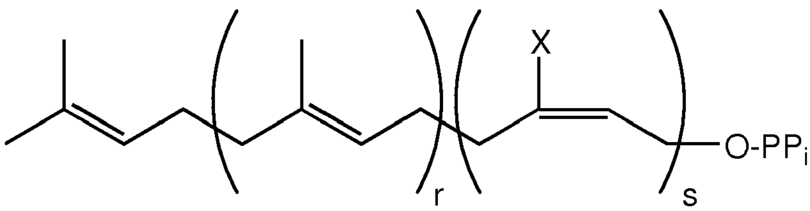

(VI),

wobei X ein Halogenatom, O-$PP_i$ eine Diphosphatgruppe oder durch O-H ersetzt ist, r eine ganze Zahl von 1 bis 6 und s eine ganze Zahl von 1 bis 30000 ist,
mit der Maßgabe, dass,

- wenn X ein Bromatom ist, s größer als 2 ist oder
- wenn X ein Chloratom ist, s größer als 1 ist.

**18.** Verwendung von Prenyltransferasen zur Herstellung von Produkten mit Molekülketten, die Monomereinheiten enthalten, die durch die Formel (II) dargestellt werden:

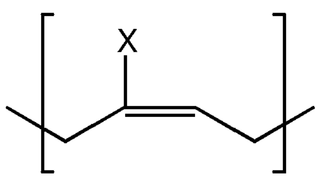

(II)

wobei X ein Halogenatom ist; und
optional, wobei die Molekülketten zusätzlich Monomereinheiten enthalten, die durch die Formel (III) dargestellt werden:

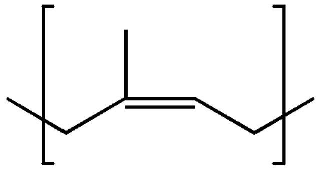

(III),

mit der Maßgabe, dass, wenn die Monomereinheiten mit der Formel (III) nicht enthalten sind

- und wenn X Brom ist, die Produkte mit Molekülketten mehr als 2 Monomereinheiten mit der Formel (II) aufweisen;
- und wenn X Chlor ist, die Produkte mit Molekülketten mehr als 1 Monomereinheit mit der Formel (II) aufweisen;

und
mit der Maßgabe, dass, wenn die Monomereinheiten mit der Formel (III) enthalten ist, der Anteil der Produkte mit Molekülketten, die mindestens eine Monomereinheit mit der Formel (II) und mindestens eine Monomereinheit mit der Formel (III) aufweisen, mindestens 0,1 Gew.-%, bevorzugt mindestens 1 Gew.-% bezogen auf das Trockengewicht beträgt.

A

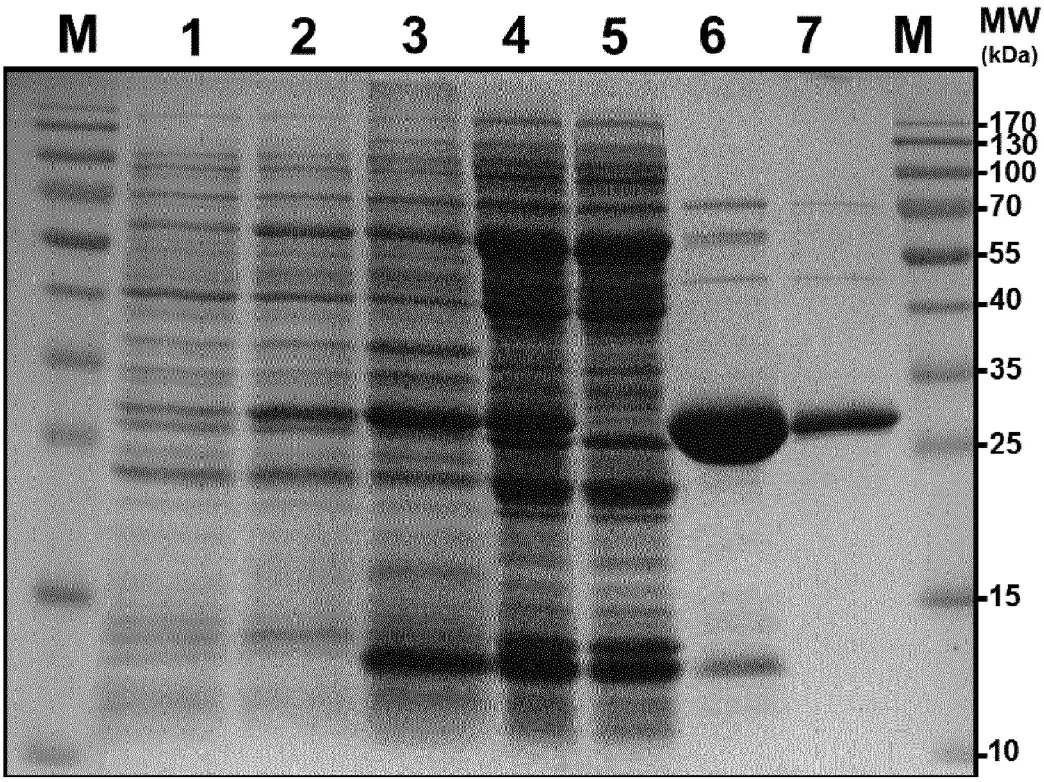


Abb. 1 (1/2)

B

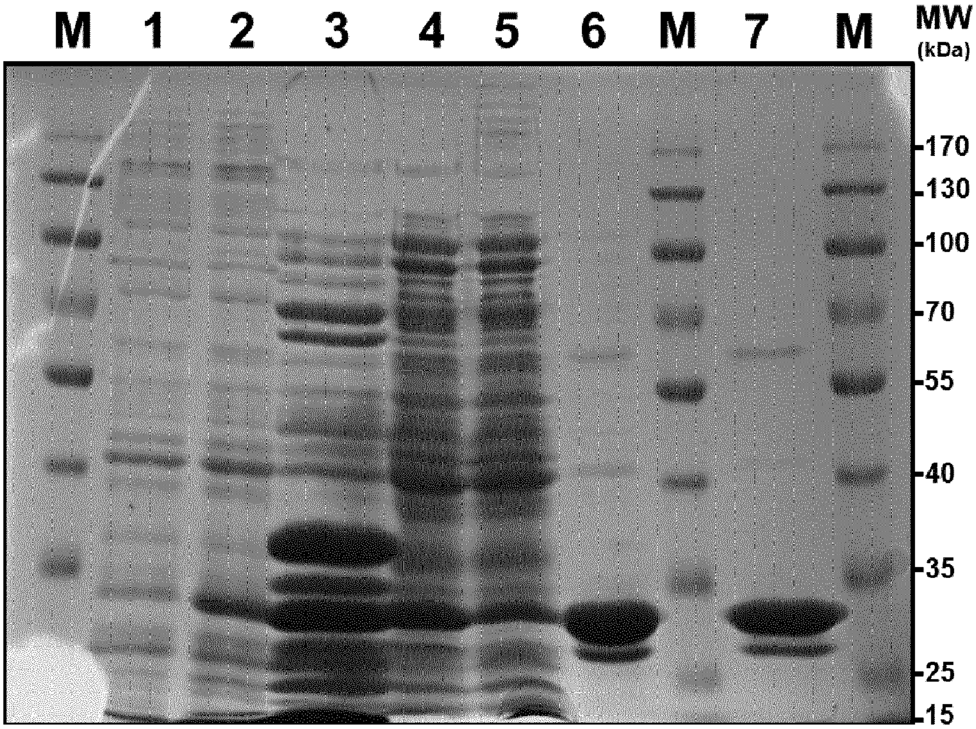


Abb. 1 (2/2)

A

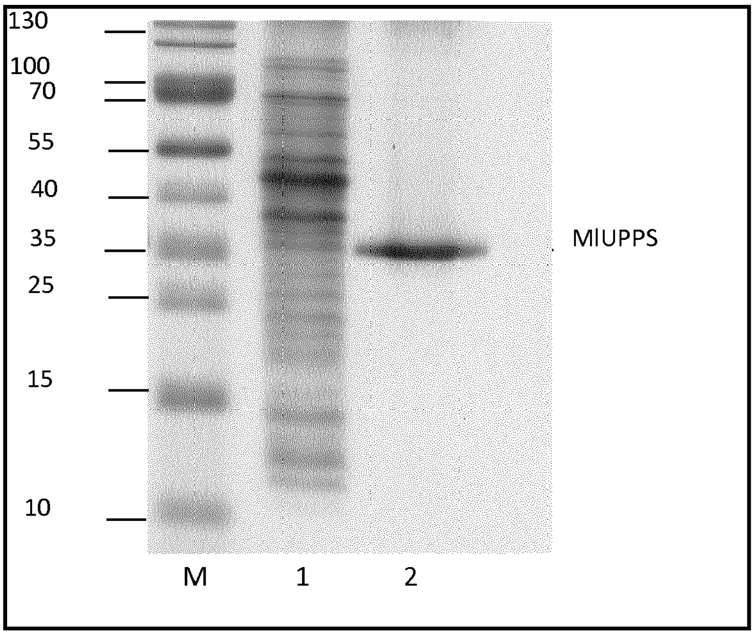
130
100
70
55
40
35
25
15
10
MlUPPS
M
1
2

B

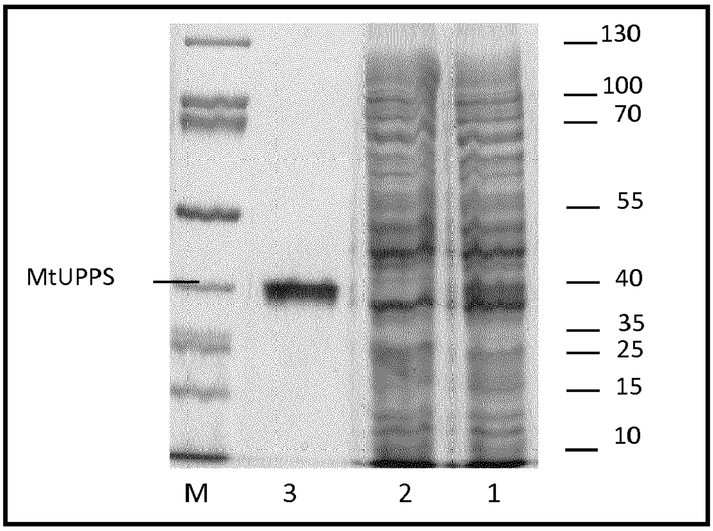
MtUPPS
130
100
70
55
40
35
25
15
10
M
3
2
1

Abb. 2 (1/2)

C

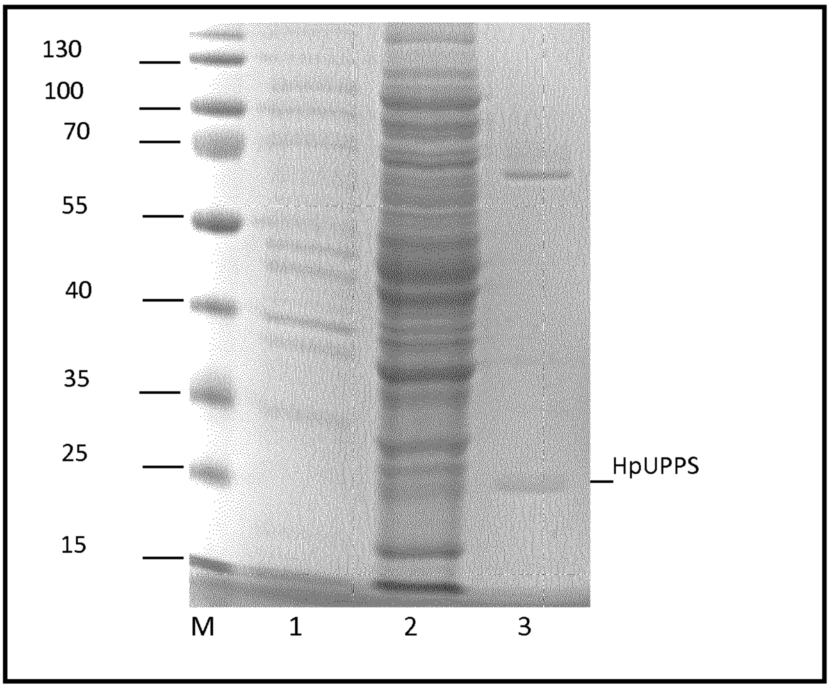


Abb. 2 (2/2)

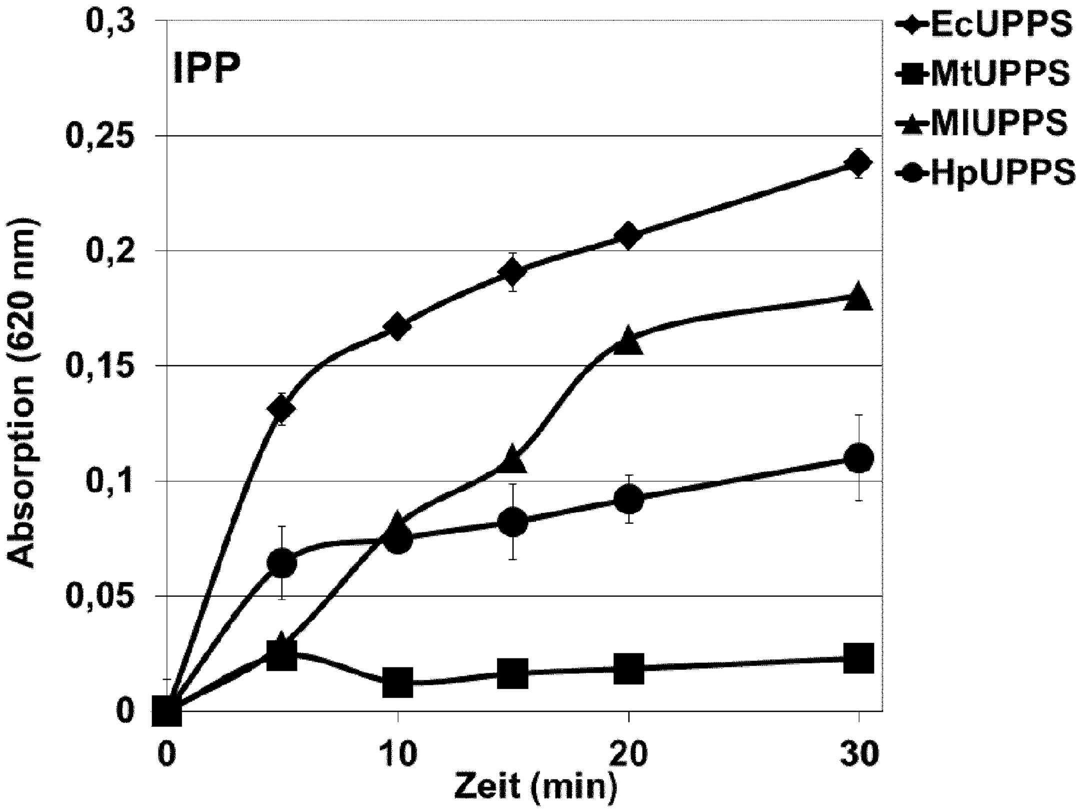
IPP
0,3
0,25
0,2
0,15
0,1
0,05
0
Absorption (620 nm)
0
10
20
30
Zeit (min)
EcUPPS
MtUPPS
MlUPPS
HpUPPS

Abb. 3

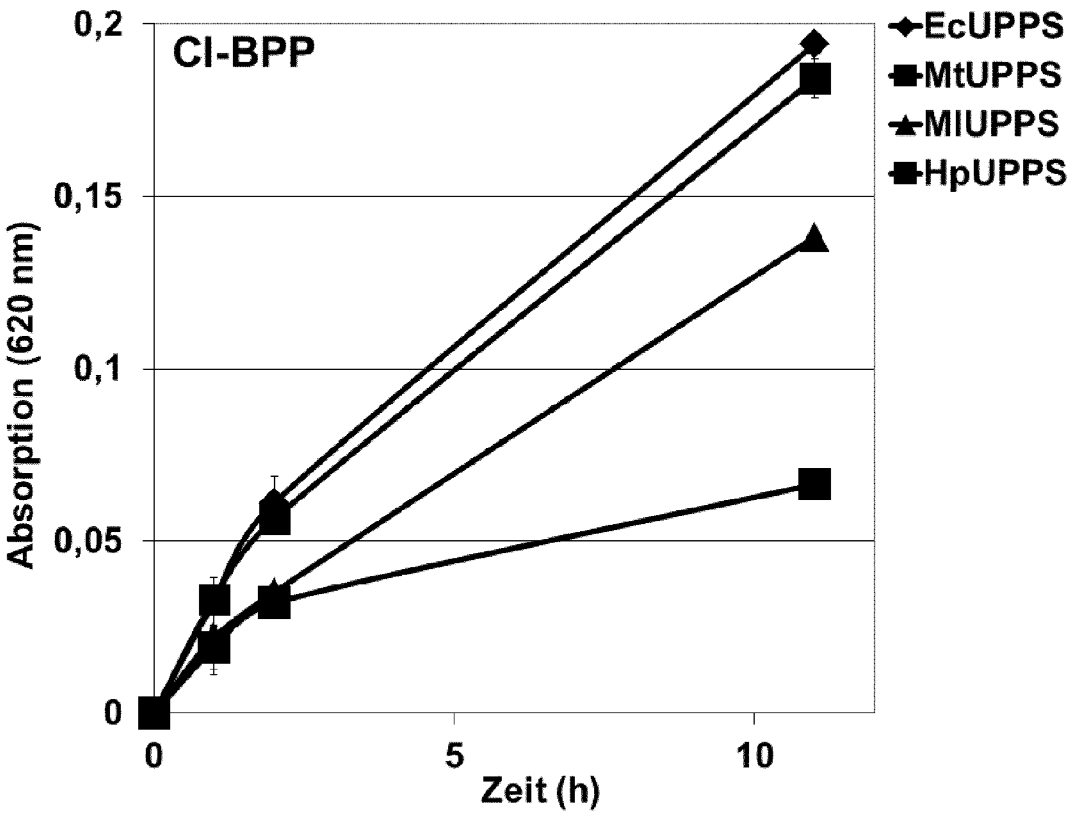
Cl-BPP
EcUPPS
MtUPPS
MlUPPS
HpUPPS
Absorption (620 nm)
0,2
0,15
0,1
0,05
0
0
5
10
Zeit (h)

Abb. 4

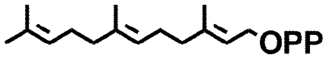

*E,E*-FPP

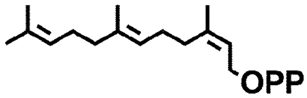

*Z,E*-FPP

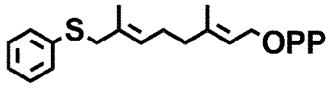

100

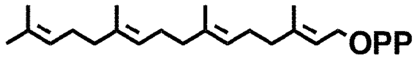

GGPP

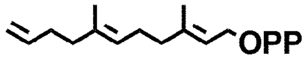

101

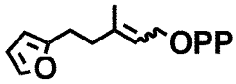

102

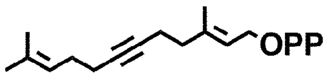

103

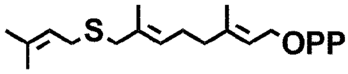

104

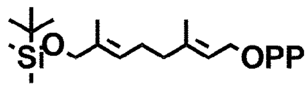

105

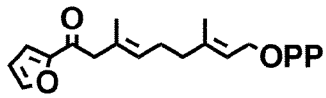

106

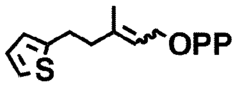

107

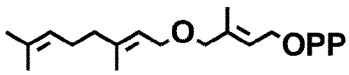

108

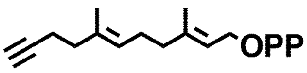

109

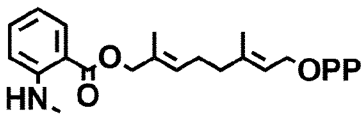

110

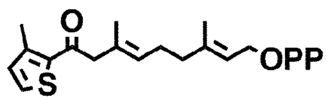

111

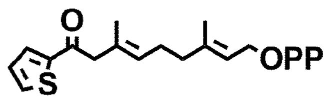

112

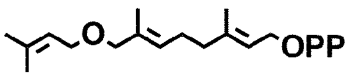

113

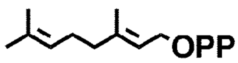

GPP

Abb. 5

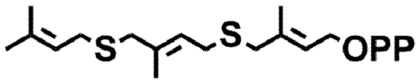 114

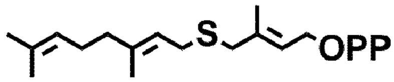 115

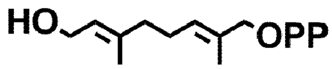 116

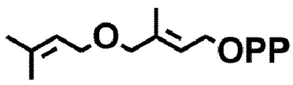 117

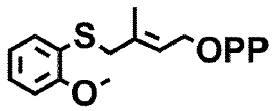 118

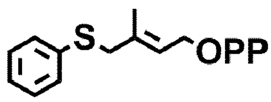 119

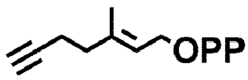 120

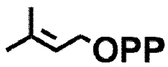 DMAPP

Abb. 6

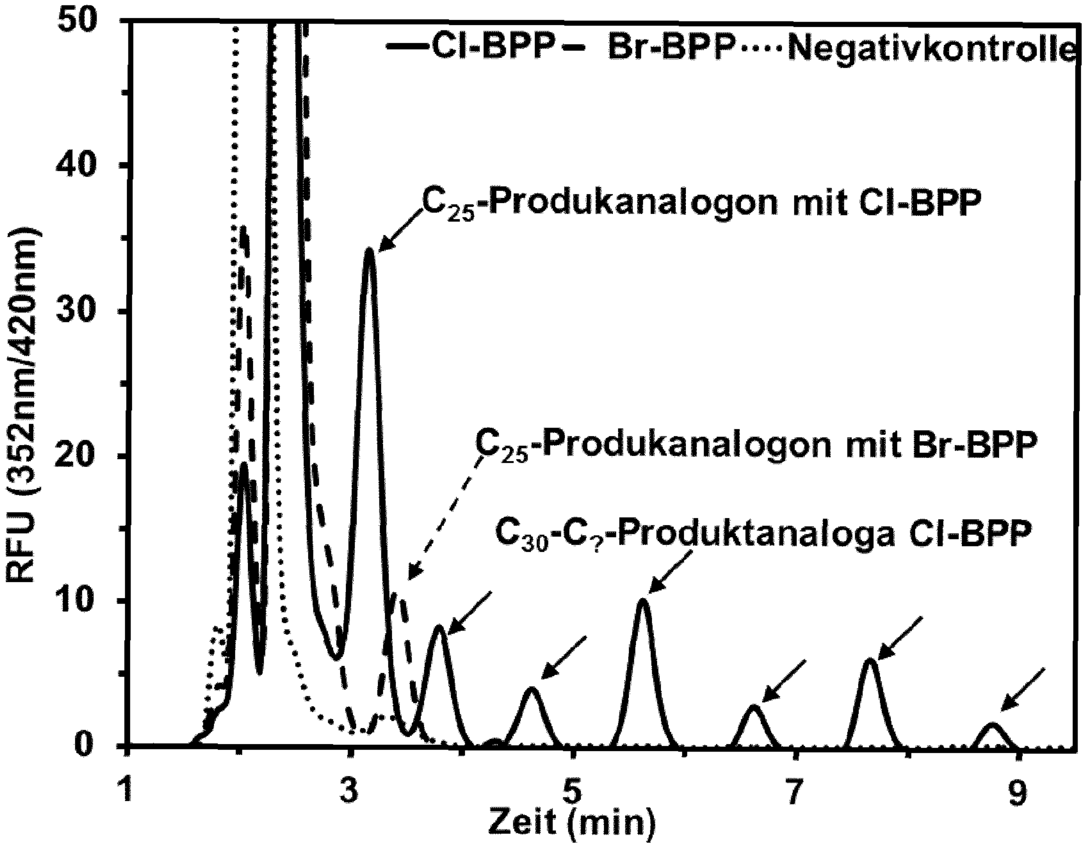

50
40
30
20
10
0
RFU (352nm/420nm)
—Cl-BPP – Br-BPP····Negativkontrolle
C25-Produkanalogon mit Cl-BPP
C25-Produkanalogon mit Br-BPP
C30-C?-Produktanaloga Cl-BPP
1
3
5
7
9
Zeit (min)


Abb. 7

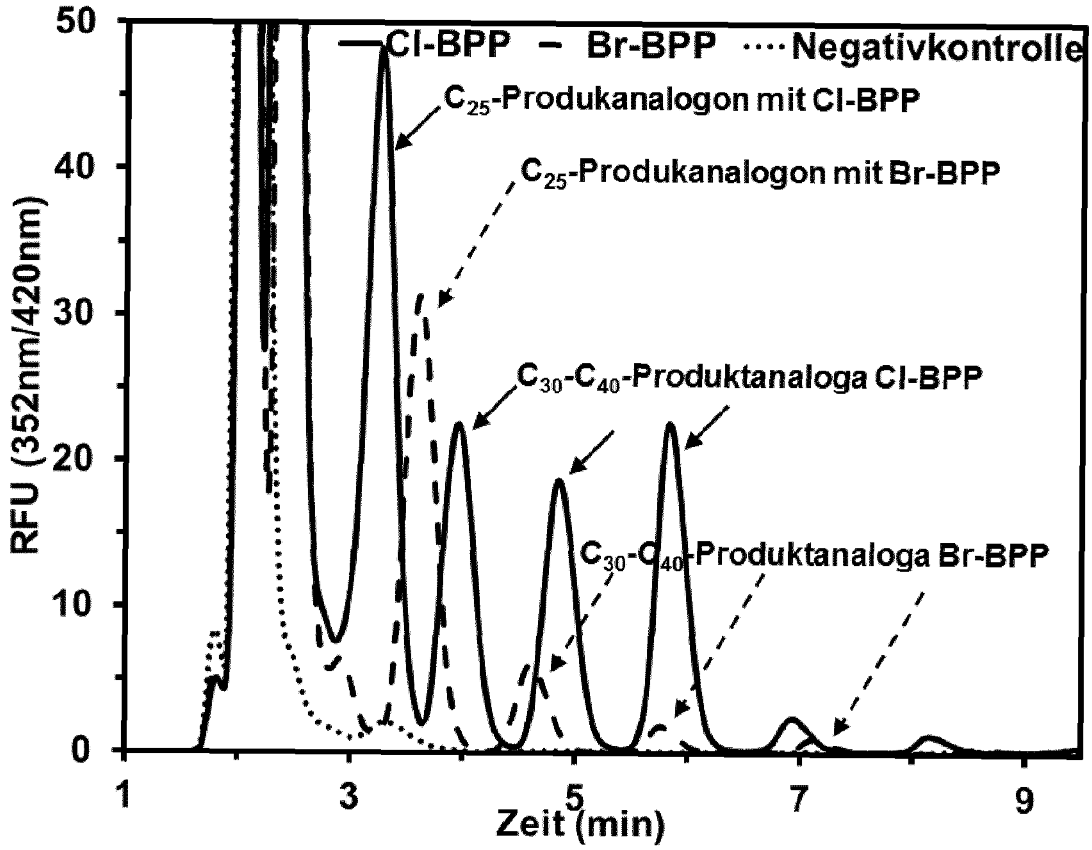

50
40
30
20
10
0
RFU (352nm/420nm)
Cl-BPP
Br-BPP
Negativkontrolle
$C_{25}$-Produkanalogon mit Cl-BPP
$C_{25}$-Produkanalogon mit Br-BPP
$C_{30}$-$C_{40}$-Produktanaloga Cl-BPP
$C_{30}$-$C_{40}$-Produktanaloga Br-BPP
1
3
5
7
9
Zeit (min)


Abb. 8

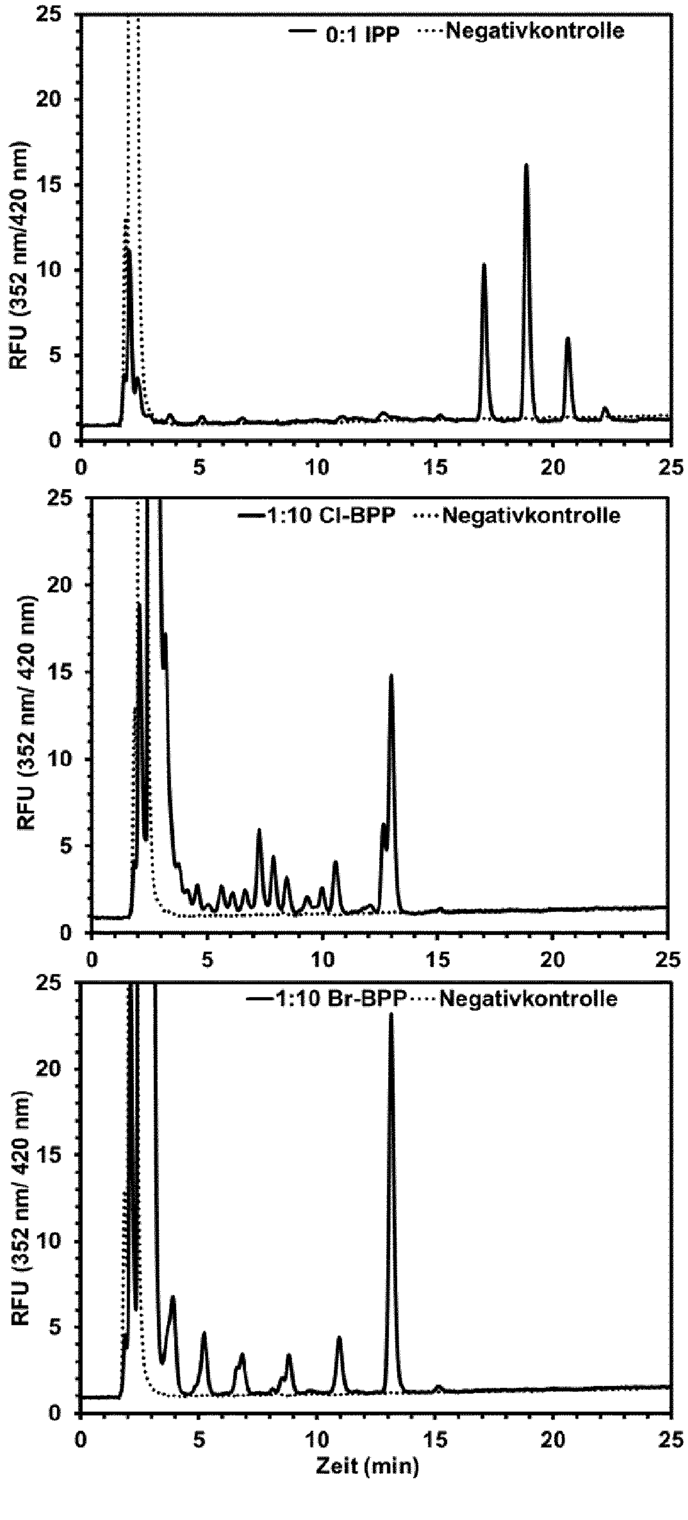
0:1 IPP
Negativkontrolle
1:10 Cl-BPP
Negativkontrolle
1:10 Br-BPP
Negativkontrolle
RFU (352 nm/420 nm)
RFU (352 nm/ 420 nm)
RFU (352 nm/ 420 nm)
Zeit (min)

Abb. 9

Europäisches Patentamt
European Patent Office
Office européen des brevets

# EUROPÄISCHER RECHERCHENBERICHT

Nummer der Anmeldung
EP 13 18 4167

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 86/06095 A1 (GOODYEAR TIRE & RUBBER [US]) 23. Oktober 1986 (1986-10-23)<br>* Ansprüche 36-51. *<br>----- | 1-18 | INV.<br>C12P5/00<br>C12P9/00 |
| Y | N.A. HEAPS ET AL: "Synthesis and Evaluation of Chlorinated Substrate Analogues for Farnesyl Diphosphate Synthase",<br>JOURNAL OF ORGANIC CHEMISTRY,<br>Bd. 76, Nr. 6, 18. März 2011 (2011-03-18), Seiten 1838-1843, XP002721057,<br>DOI: 10.1021/jo1024305<br>* Zusammenfassung *<br>* Seite 1840; Abbildung 3 *<br>----- | 1-18 | |
| A | YAMADA Y ET AL: "Efficient in vitro synthesis of cis-polyisoprenes using a thermostable cis-prenyltransferase from a hyperthermophilic archaeon Thermococcus kodakaraensis",<br>JOURNAL OF BIOTECHNOLOGY, ELSEVIER SCIENCE PUBLISHERS, AMSTERDAM, NL,<br>Bd. 143, Nr. 2,<br>20. August 2009 (2009-08-20), Seiten 151-156, XP026467238,<br>ISSN: 0168-1656, DOI: 10.1016/J.JBIOTEC.2009.06.026<br>[gefunden am 2009-07-05]<br>* Zusammenfassung *<br>* Seite 152; Abbildung 1 *<br>----- | 1-18 | RECHERCHIERTE SACHGEBIETE (IPC)<br>C12P |
| L | EP 2 594 597 A1 (SUMITOMO RUBBER IND [JP]; UNIV YAMAGATA [JP])<br>22. Mai 2013 (2013-05-22) | 1-18 | |
| A | & WO 2012/008298 A1 (SUMITOMO RUBBER IND LTD) 19. Januar 2012 (2012-01-19)<br>* das ganze Dokument *<br>----- | 1-18 | |

2

Der vorliegende Recherchenbericht wurde für alle Patentansprüche erstellt

| Recherchenort | Abschlußdatum der Recherche | Prüfer |
|---|---|---|
| Berlin | 3. März 2014 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 13 18 4167

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-03-2014

| Im Recherchenbericht angeführtes Patentdokument | Datum der Veröffentlichung | Mitglied(er) der Patentfamilie | Datum der Veröffentlichung |
|---|---|---|---|
| WO 8606095 A1 | 23-10-1986 | AU 601147 B2 | 06-09-1990 |
| | | AU 5581286 A | 05-11-1986 |
| | | AU 5876390 A | 25-10-1990 |
| | | BR 8606536 A | 04-08-1987 |
| | | CN 86102341 A | 15-10-1986 |
| | | CN 88100484 A | 10-08-1988 |
| | | EP 0216855 A1 | 08-04-1987 |
| | | IL 78337 A | 15-04-1991 |
| | | IN 170383 A1 | 21-03-1992 |
| | | JP S62502797 A | 12-11-1987 |
| | | US 4638028 A | 20-01-1987 |
| | | WO 8606095 A1 | 23-10-1986 |
| | | ZA 8601928 A | 29-10-1986 |
| EP 2594597 A1 | 22-05-2013 | CN 103025785 A | 03-04-2013 |
| | | EP 2594597 A1 | 22-05-2013 |
| | | JP 5058332 B2 | 24-10-2012 |
| | | JP 2012036360 A | 23-02-2012 |
| | | KR 20130043172 A | 29-04-2013 |
| | | WO 2012008298 A1 | 19-01-2012 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **W.H. CAROTHERS.** *J. Am. Chem. Soc.,* 1929, vol. 51, 2548-2559

SEQUENCE LISTING

<110>  Lanxess Deutschland GmbH

<120>  Verkettung von halogenierten Alkenyldiphosphat-Derivaten

<130>  P60385EP

<160>  9

<170>  PatentIn version 3.3

<210>  1
<211>  253
<212>  PRT
<213>  Escherichia coli

<400>  1

```
Met Met Leu Ser Ala Thr Gln Pro Leu Ser Glu Lys Leu Pro Ala His
1               5                   10                  15

Gly Cys Arg His Val Ala Ile Ile Met Asp Gly Asn Gly Arg Trp Ala
            20                  25                  30

Lys Lys Gln Gly Lys Ile Arg Ala Phe Gly His Lys Ala Gly Ala Lys
        35                  40                  45

Ser Val Arg Arg Ala Val Ser Phe Ala Ala Asn Asn Gly Ile Glu Ala
    50                  55                  60

Leu Thr Leu Tyr Ala Phe Ser Ser Glu Asn Trp Asn Arg Pro Ala Gln
65                  70                  75                  80

Glu Val Ser Ala Leu Met Glu Leu Phe Val Trp Ala Leu Asp Ser Glu
                85                  90                  95

Val Lys Ser Leu His Arg His Asn Val Arg Leu Arg Ile Ile Gly Asp
            100                 105                 110

Thr Ser Arg Phe Asn Ser Arg Leu Gln Glu Arg Ile Arg Lys Ser Glu
        115                 120                 125

Ala Leu Thr Ala Gly Asn Thr Gly Leu Thr Leu Asn Ile Ala Ala Asn
    130                 135                 140

Tyr Gly Gly Arg Trp Asp Ile Val Gln Gly Val Arg Gln Leu Ala Glu
145                 150                 155                 160

Lys Val Gln Gln Gly Asn Leu Gln Pro Asp Gln Ile Asp Glu Glu Met
                165                 170                 175
```

```
Leu Asn Gln His Val Cys Met His Glu Leu Ala Pro Val Asp Leu Val
            180                 185                 190

Ile Arg Thr Gly Gly Glu His Arg Ile Ser Asn Phe Leu Leu Trp Gln
        195                 200                 205

Ile Ala Tyr Ala Glu Leu Tyr Phe Thr Asp Val Leu Trp Pro Asp Phe
    210                 215                 220

Asp Glu Gln Asp Phe Glu Gly Ala Leu Asn Ala Phe Ala Asn Arg Glu
225                 230                 235                 240

Arg Arg Phe Gly Gly Thr Glu Pro Gly Asp Glu Thr Ala
                245                 250


<210>  2
<211>  264
<212>  PRT
<213>  Thermococcus kodakaraensis

<400>  2

Met Leu Tyr Arg Leu Val Ser His Ile Pro His Ile Leu Phe Lys Pro
1               5                   10                  15

Val Tyr Asp Ala Tyr Glu Ser Tyr Leu Leu Glu Lys Val Lys Ser Gly
            20                  25                  30

Asn Ile Pro Lys His Val Ala Ile Ile Met Asp Gly Asn Arg Arg Trp
        35                  40                  45

Ala Arg Lys Leu Glu Lys Pro Pro Trp Tyr Gly His Leu Phe Gly Ser
    50                  55                  60

Lys Lys Leu Glu Glu Ile Leu Glu Trp Cys Arg Glu Leu Asn Ile Arg
65                  70                  75                  80

Thr Leu Thr Val Tyr Ala Phe Ser Thr Glu Asn Phe Lys Arg Ser Lys
                85                  90                  95

Glu Glu Val Glu Ala Leu Met Asn Leu Phe Glu Glu Lys Phe Lys Glu
            100                 105                 110

Leu Val Gln Asp Glu Arg Val His Arg Tyr Gly Ile Arg Val Asn Val
        115                 120                 125

Leu Gly Arg Lys Asp Met Leu Pro Glu Asn Val Arg Lys Ala Ala Glu
    130                 135                 140
```

```
Glu Ala Glu Arg Ala Thr Arg Lys Tyr Ser Asn Tyr Asn Leu Asn Ile
145                 150                 155                 160

Ala Leu Ala Tyr Gly Gly Arg Ser Glu Ile Ala Asp Ala Val Arg Glu
                165                 170                 175

Ile Val Arg Asp Val Leu Glu Gly Lys Ile Lys Pro Glu Asp Ile Asp
            180                 185                 190

Glu Glu Ala Ile Lys Arg Tyr Leu Tyr Tyr Pro Asn Met Pro Asp Pro
        195                 200                 205

Asp Ile Val Ile Arg Thr Gly Gly Glu Val Arg Ile Ser Asn Phe Leu
    210                 215                 220

Leu Tyr Gln Ile Ala Tyr Ser Glu Leu Phe Phe Val Asp Val Tyr Phe
225                 230                 235                 240

Pro Glu Phe Arg Lys Ile Asp Phe Leu Arg Ile Ile Arg Glu Phe Gln
                245                 250                 255

Lys Arg Gln Arg Arg Phe Gly Arg
            260


<210>  3
<211>  249
<212>  PRT
<213>  Micrococcus luteus

<400>  3

Met Phe Pro Ile Lys Lys Arg Lys Ala Ile Lys Asn Asn Asn Ile Asn
1               5                   10                  15

Ala Ala Gln Ile Pro Lys His Ile Ala Ile Ile Met Asp Gly Asn Gly
            20                  25                  30

Arg Trp Ala Lys Gln Lys Lys Met Pro Arg Ile Lys Gly His Tyr Glu
        35                  40                  45

Gly Met Gln Thr Val Lys Lys Ile Thr Arg Tyr Ala Ser Asp Leu Gly
    50                  55                  60

Val Lys Tyr Leu Thr Leu Tyr Ala Phe Ser Thr Glu Asn Trp Ser Arg
65                  70                  75                  80

Pro Lys Asp Glu Val Asn Tyr Leu Met Lys Leu Pro Gly Asp Phe Leu
                85                  90                  95
```

```
Asn Thr Phe Leu Pro Glu Leu Ile Glu Lys Asn Val Lys Val Glu Thr
            100                 105                 110

Ile Gly Phe Ile Asp Asp Leu Pro Asp His Thr Lys Lys Ala Val Leu
        115                 120                 125

Glu Ala Lys Glu Lys Thr Lys His Asn Thr Gly Leu Thr Leu Val Phe
    130                 135                 140

Ala Leu Asn Tyr Gly Gly Arg Lys Glu Ile Ile Ser Ala Val Gln Leu
145                 150                 155                 160

Ile Ala Glu Arg Tyr Lys Ser Gly Glu Ile Ser Leu Asp Glu Ile Ser
                165                 170                 175

Glu Thr His Phe Asn Glu Tyr Leu Phe Thr Ala Asn Met Pro Asp Pro
            180                 185                 190

Glu Leu Leu Ile Arg Thr Ser Gly Glu Glu Arg Leu Ser Asn Phe Leu
        195                 200                 205

Ile Trp Gln Cys Ser Tyr Ser Glu Phe Val Phe Ile Asp Glu Phe Trp
    210                 215                 220

Pro Asp Phe Asn Glu Glu Ser Leu Ala Gln Cys Ile Ser Ile Tyr Gln
225                 230                 235                 240

Asn Arg His Arg Arg Phe Gly Gly Leu
                245


<210>  4
<211>  296
<212>  PRT
<213>  Mycobacterium tuberculosis

<400>  4

Met Ala Arg Asp Ala Arg Lys Arg Thr Ser Ser Asn Phe Pro Gln Leu
1               5                   10                  15

Pro Pro Ala Pro Asp Asp Tyr Pro Thr Phe Pro Asp Thr Ser Thr Trp
            20                  25                  30

Pro Val Val Phe Pro Glu Leu Pro Ala Ala Pro Tyr Gly Gly Pro Cys
        35                  40                  45

Arg Pro Pro Gln His Thr Ser Lys Ala Ala Ala Pro Arg Ile Pro Ala
    50                  55                  60
```

```
Asp Arg Leu Pro Asn His Val Ala Ile Val Met Asp Gly Asn Gly Arg
65                  70                  75                  80

Trp Ala Thr Gln Arg Gly Leu Ala Arg Thr Glu Gly His Lys Met Gly
                85                  90                  95

Glu Ala Val Val Ile Asp Ile Ala Cys Gly Ala Ile Glu Leu Gly Ile
            100                 105                 110

Lys Trp Leu Ser Leu Tyr Ala Phe Ser Thr Glu Asn Trp Lys Arg Ser
        115                 120                 125

Pro Glu Glu Val Arg Phe Leu Met Gly Phe Asn Arg Asp Val Val Arg
    130                 135                 140

Arg Arg Arg Asp Thr Leu Lys Lys Leu Gly Val Arg Ile Arg Trp Val
145                 150                 155                 160

Gly Ser Arg Pro Arg Leu Trp Arg Ser Val Ile Asn Glu Leu Ala Val
                165                 170                 175

Ala Glu Glu Met Thr Lys Ser Asn Asp Val Ile Thr Ile Asn Tyr Cys
            180                 185                 190

Val Asn Tyr Gly Gly Arg Thr Glu Ile Thr Glu Ala Thr Arg Glu Ile
        195                 200                 205

Ala Arg Glu Val Ala Ala Gly Arg Leu Asn Pro Glu Arg Ile Thr Glu
    210                 215                 220

Ser Thr Ile Ala Arg His Leu Gln Arg Pro Asp Ile Pro Asp Val Asp
225                 230                 235                 240

Leu Phe Leu Arg Thr Ser Gly Glu Gln Arg Ser Ser Asn Phe Met Leu
                245                 250                 255

Trp Gln Ala Ala Tyr Ala Glu Tyr Ile Phe Gln Asp Lys Leu Trp Pro
            260                 265                 270

Asp Tyr Asp Arg Arg Asp Leu Trp Ala Ala Cys Glu Glu Tyr Ala Ser
        275                 280                 285

Arg Thr Arg Arg Phe Gly Ser Ala
    290                 295

<210>  5
<211>  234
<212>  PRT
```

<213>  Helicobacter pylori

<400>  5

```
Met Asp Asn Thr Leu Lys His Leu Ala Ile Ile Met Asp Gly Asn Gly
1               5                   10                  15

Arg Trp Ala Lys Leu Lys Asn Lys Ala Arg Ala Tyr Gly His Lys Lys
            20                  25                  30

Gly Val Lys Thr Leu Lys Asp Ile Thr Ile Trp Cys Ala Asn His Lys
        35                  40                  45

Leu Glu Cys Leu Thr Leu Tyr Ala Phe Ser Thr Glu Asn Trp Lys Arg
    50                  55                  60

Pro Lys Ser Glu Val Asp Phe Leu Met Lys Met Leu Lys Lys Tyr Leu
65                  70                  75                  80

Lys Asp Glu Arg Ser Thr Tyr Leu Asn Asn Asn Ile Arg Phe Arg Ala
                85                  90                  95

Ile Gly Asp Leu Glu Gly Phe Ser Lys Glu Leu Arg Asp Thr Ile Leu
            100                 105                 110

Gln Leu Glu Asn Asp Thr Arg His Phe Lys Asp Phe Thr Gln Val Leu
        115                 120                 125

Ala Leu Asn Tyr Gly Ser Lys Asn Glu Leu Ser Arg Ala Phe Lys Ser
    130                 135                 140

Leu Leu Glu Ser Pro Pro Ser His Ile Asn Leu Leu Glu Ser Leu Glu
145                 150                 155                 160

Asn Glu Ile Ser Asn Arg Leu Asp Thr His Asp Leu Pro Glu Val Asp
                165                 170                 175

Leu Leu Leu Arg Thr Gly Gly Glu Met Arg Leu Ser Asn Phe Leu Leu
            180                 185                 190

Trp Gln Ser Ser Tyr Ala Glu Leu Phe Phe Thr Pro Ile Leu Trp Pro
        195                 200                 205

Asp Phe Thr Pro Lys Asp Leu Glu Asn Ile Ile Ser Asp Phe Tyr Lys
    210                 215                 220

Arg Val Arg Lys Phe Gly Glu Leu Lys Cys
225                 230
```

<210> 6
<211> 333
<212> PRT
<213> Homo sapiens

<400> 6

```
Met Ser Trp Ile Lys Glu Gly Glu Leu Ser Leu Trp Glu Arg Phe Cys
1               5                   10                  15

Ala Asn Ile Ile Lys Ala Gly Pro Met Pro Lys His Ile Ala Phe Ile
            20                  25                  30

Met Asp Gly Asn Arg Arg Tyr Ala Lys Lys Cys Gln Val Glu Arg Gln
        35                  40                  45

Glu Gly His Ser Gln Gly Phe Asn Lys Leu Ala Glu Thr Leu Arg Trp
    50                  55                  60

Cys Leu Asn Leu Gly Ile Leu Glu Val Thr Val Tyr Ala Phe Ser Ile
65                  70                  75                  80

Glu Asn Phe Lys Arg Ser Lys Ser Glu Val Asp Gly Leu Met Asp Leu
                85                  90                  95

Ala Arg Gln Lys Phe Ser Arg Leu Met Glu Glu Lys Glu Lys Leu Gln
            100                 105                 110

Lys His Gly Val Cys Ile Arg Val Leu Gly Asp Leu His Leu Leu Pro
        115                 120                 125

Leu Asp Leu Gln Glu Leu Ile Ala Gln Ala Val Gln Ala Thr Lys Asn
    130                 135                 140

Tyr Asn Lys Cys Phe Leu Asn Val Cys Phe Ala Tyr Thr Ser Arg His
145                 150                 155                 160

Glu Ile Ser Asn Ala Val Arg Glu Met Ala Trp Gly Val Glu Gln Gly
                165                 170                 175

Leu Leu Asp Pro Ser Asp Ile Ser Glu Ser Leu Leu Asp Lys Cys Leu
            180                 185                 190

Tyr Thr Asn Arg Ser Pro His Pro Asp Ile Leu Ile Arg Thr Ser Gly
        195                 200                 205

Glu Val Arg Leu Ser Asp Phe Leu Leu Trp Gln Thr Ser His Ser Cys
    210                 215                 220
```

Leu Val Phe Gln Pro Val Leu Trp Pro Glu Tyr Thr Phe Trp Asn Leu
225 230 235 240

Phe Glu Ala Ile Leu Gln Phe Gln Met Asn His Ser Val Leu Gln Lys
245 250 255

Ala Arg Asp Met Tyr Ala Glu Glu Arg Lys Arg Gln Gln Leu Glu Arg
260 265 270

Asp Gln Ala Thr Val Thr Glu Gln Leu Leu Arg Glu Gly Leu Gln Ala
275 280 285

Ser Gly Asp Ala Gln Leu Arg Arg Thr Arg Leu His Lys Leu Ser Ala
290 295 300

Arg Arg Glu Glu Arg Val Gln Gly Phe Leu Gln Ala Leu Glu Leu Lys
305 310 315 320

Arg Ala Asp Trp Leu Ala Arg Leu Gly Thr Ala Ser Ala
325 330

<210> 7
<211> 284
<212> PRT
<213> Hevea brasiliensis

<400> 7

Met Glu Leu Tyr Asn Gly Glu Arg Pro Ser Val Phe Arg Leu Leu Gly
1 5 10 15

Lys Tyr Met Arg Lys Gly Leu Tyr Ser Ile Leu Thr Gln Gly Pro Ile
20 25 30

Pro Thr His Ile Ala Phe Ile Leu Asp Gly Asn Gly Arg Phe Ala Lys
35 40 45

Lys His Lys Leu Pro Glu Gly Gly Gly His Lys Ala Gly Phe Leu Ala
50 55 60

Leu Leu Asn Val Leu Thr Tyr Cys Tyr Glu Leu Gly Val Lys Tyr Ala
65 70 75 80

Thr Ile Tyr Ala Phe Ser Ile Asp Asn Phe Arg Arg Lys Pro His Glu
85 90 95

Val Gln Tyr Val Met Asn Leu Met Leu Glu Lys Ile Glu Gly Met Ile
100 105 110

```
Met Glu Glu Ser Ile Ile Asn Ala Tyr Asp Ile Cys Val Arg Phe Val
        115                 120                 125

Gly Asn Leu Lys Leu Leu Asp Glu Pro Leu Lys Thr Ala Ala Asp Lys
    130                 135                 140

Ile Met Arg Ala Thr Ala Lys Asn Ser Lys Phe Val Leu Leu Leu Ala
145                 150                 155                 160

Val Cys Tyr Thr Ser Thr Asp Glu Ile Val His Ala Val Glu Glu Ser
                165                 170                 175

Ser Lys Asp Lys Leu Lys Ser Asp Glu Ile Cys Asn Asp Gly Asn Gly
            180                 185                 190

Asp Cys Val Ile Lys Ile Glu Glu Met Glu Pro Tyr Ser Glu Ile Lys
        195                 200                 205

Leu Val Glu Leu Glu Arg Asn Thr Tyr Ile Asn Pro Tyr Pro Asp Val
    210                 215                 220

Leu Ile Arg Thr Ser Gly Glu Thr Arg Leu Ser Asn Tyr Leu Leu Trp
225                 230                 235                 240

Gln Thr Thr Asn Cys Ile Leu Tyr Ser Pro His Ala Leu Trp Pro Glu
                245                 250                 255

Ile Gly Leu Arg His Val Val Trp Ala Val Ile Asn Cys Gln Arg His
            260                 265                 270

Tyr Ser Tyr Leu Glu Lys His Lys Glu Tyr Leu Lys
        275                 280


<210>  8
<211>  308
<212>  PRT
<213>  Taraxacum koksaghyz

<400>  8

Met Gln Val Asn Pro Ile Ile Thr Thr Asp Ser Ser Leu Lys Leu Val
1               5                   10                  15

Glu Glu Glu Arg Ser Asn Gly Arg Ile Gly Asn Phe Leu Gly Gly Leu
            20                  25                  30

Asn Ala Thr Leu Arg Lys Leu Val Phe Arg Val Ile Ala Ser Arg Pro
        35                  40                  45
```

```
Ile Pro Glu His Ile Ala Phe Ile Leu Asp Gly Asn Arg Arg Phe Ala
    50                  55                  60

Arg Lys Trp Asn Leu Thr Glu Gly Thr Gly His Lys Thr Gly Phe Leu
65                  70                  75                  80

Ala Leu Met Ser Val Leu Lys Tyr Cys Tyr Glu Ile Gly Val Lys Tyr
                85                  90                  95

Val Thr Ile Tyr Ala Phe Ser Leu Asp Asn Phe Asn Arg Arg Pro Asp
            100                 105                 110

Glu Val Gln Tyr Val Met Asp Leu Met Gln Asp Lys Ile Glu Gly Phe
        115                 120                 125

Leu Lys Glu Val Ser Ile Ile Asn Gln Tyr Gly Val Arg Val Leu Phe
    130                 135                 140

Ile Gly Asp Leu Asp Arg Leu Tyr Glu Pro Val Arg Ile Ala Ala Glu
145                 150                 155                 160

Lys Ala Met Glu Ala Thr Ala Lys Asn Ser Thr Thr Tyr Leu Leu Val
                165                 170                 175

Cys Val Ala Tyr Thr Ser Ser His Glu Ile Pro Arg Ala Ile His Glu
            180                 185                 190

Ala Cys Glu Glu Lys Ser Gly Ala Met Ala Asn Ser Ile Arg Val Met
        195                 200                 205

Asn Gly Asn Gly Phe Phe Asn Gly Asn Gly Tyr Thr Asn Val Asn His
    210                 215                 220

Gly Ser Gln Ala Val Ile Lys Val Val Asp Leu Asp Lys His Met Tyr
225                 230                 235                 240

Met Gly Val Ala Pro Asp Pro Asp Ile Leu Val Arg Ser Ser Gly Glu
                245                 250                 255

Thr Arg Leu Ser Asn Phe Leu Leu Trp Gln Thr Thr Asn Cys Leu Leu
            260                 265                 270

Tyr Ser Pro Lys Ala Leu Trp Pro Glu Met Gly Phe Trp Gln Val Val
        275                 280                 285

Trp Gly Ile Leu Glu Phe Gln Asn Asn Tyr Asn Tyr Leu Glu Lys Lys
    290                 295                 300
```

```
Lys Lys Gln Ala
305


<210>  9
<211>  303
<212>  PRT
<213>  Arabidopsis thaliana

<400>  9

Met Leu Ser Leu Leu Ser Ser Asp Ser Ser Leu Leu Ser Leu Leu Phe
1               5                   10                  15


Leu Phe Leu Ile Pro Cys Leu Phe Ile Thr Ser Tyr Ile Gly Phe Pro
            20                  25                  30


Val Phe Leu Leu Lys Leu Ile Gly Leu Ile Lys Ile Lys Ala Ala Arg
        35                  40                  45


Asp Asn Glu Lys Arg Asp Glu Gly Thr Tyr Val Val Arg Glu Asp Gly
    50                  55                  60


Leu Gln Arg Glu Leu Met Pro Arg His Val Ala Phe Ile Leu Asp Gly
65                  70                  75                  80


Asn Arg Arg Trp Ala Lys Arg Ala Gly Leu Thr Thr Ser Gln Gly His
                85                  90                  95


Glu Ala Gly Ala Lys Arg Leu Ile Asp Ile Ala Glu Leu Cys Phe Glu
            100                 105                 110


Leu Gly Val His Thr Val Ser Ala Phe Ala Phe Ser Thr Glu Asn Trp
        115                 120                 125


Gly Arg Asp Lys Ile Glu Ile Asp Asn Leu Met Ser Leu Ile Gln His
    130                 135                 140


Tyr Arg Asn Lys Ser Asn Ile Lys Phe Phe His Arg Ser Glu Val Arg
145                 150                 155                 160


Val Ser Val Ile Gly Asn Lys Thr Lys Ile Pro Glu Ser Leu Leu Lys
                165                 170                 175


Glu Ile His Glu Ile Glu Glu Ala Thr Lys Gly Tyr Lys Asn Lys His
            180                 185                 190


Leu Ile Met Ala Val Asp Tyr Ser Gly Lys Phe Asp Ile Met His Ala
        195                 200                 205
```

```
Cys Lys Ser Leu Val Lys Lys Ser Glu Lys Gly Leu Ile Arg Glu Glu
    210                 215                 220

Asp Val Asp Glu Ala Leu Ile Glu Arg Glu Leu Leu Thr Asn Cys Ser
225                 230                 235                 240

Asp Phe Pro Ser Pro Asp Leu Met Ile Arg Thr Ser Gly Glu Gln Arg
                245                 250                 255

Ile Ser Asn Phe Phe Leu Trp Gln Leu Ala Tyr Ser Glu Leu Phe Phe
            260                 265                 270

Ser Pro Val Phe Trp Pro Asp Phe Asp Lys Asp Lys Leu Leu Glu Ala
        275                 280                 285

Leu Ala Ser Tyr Gln Arg Arg Glu Arg Arg Phe Gly Cys Arg Val
    290                 295                 300
```